# EUROPEAN PATENT SPECIFICATION

(11) **EP 2 259 067 B1**
(45) Date of publication and mention of the grant of the patent: **27.06.2012**
(21) Application number: 10181177.6
(22) Date of filing: 02.10.2002
(51) Int. Cl.: G01N 33/58, G01N 33/532, G01N 33/68

(54) **Methods for immunolabeling**
Immunmarkierungsverfahren
Procédés d'immunomarquage

(30) Priority: 12.10.2001 US 329068 P; 01.04.2002 US 369418 P; 05.04.2002 US 118204
(43) Date of publication of application: 08.12.2010
(62) Divisional of application: 09163588.8
(73) Proprietor: Life Technologies Corporation, Carlsbad, CA 92008 (US)
(72) Inventor: Haugland, Richard, P., Eugene, OR 87408 (US); Archer, Robert, A., Eugene, OR 97404 (US); Beechem, Joseph, M., Eugene, OR 97405 (US); Hagen, David, C., Eugene, OR 97405 (US); Haugland, Rosaria, P., Eugene, OR 97408 (US)
(74) Representative: Irvine, Jonquil Claire

(56) References cited:
- US-A- 4 481 298
- US-A- 5 281 521
- US-A- 5 585 243
- VAN DER LOOS CHRIS M ET AL: "The Animal Research Kit (ARK) can be used in a multistep double staining method for human tissue specimens", JOURNAL OF HISTOCHEMISTRY AND CYTOCHEMISTRY, vol. 48, no. 10, October 2000 (2000-10), pages 1431-1437, XP002357555, ISSN: 0022-1554
- NEGOESCU ADRIEN ET AL: "F(ab) secondary antibodies: A general method for double immunolabeling with primary antisera from the same species. Efficiency control by chemiluminescence", JOURNAL OF HISTOCHEMISTRY AND CYTOCHEMISTRY, vol. 42, no. 3, 1994, pages 433-437, XP002386335, ISSN: 0022-1554
- BROWN JEREMY K ET AL: "Primary antibody-Fab fragment complexes: A flexible alternative to traditional direct and indirect Immunolabeling techniques", JOURNAL OF HISTOCHEMISTRY & CYTOCHEMISTRY, vol. 52, no. 9, September 2004 (2004-09), pages 1219-1230, XP002357557, ISSN: 0022-1554
- INO HIDETOSHI: "Application of antigen retrieval by heating for double-label fluorescent immunohistochemistry with identical species-derived primary antibodies", JOURNAL OF HISTOCHEMISTRY & CYTOCHEMISTRY, vol. 52, no. 9, September 2004 (2004-09), pages 1209-1217, XP002387586, ISSN: 0022-1554

## Description

### Field of the Invention

The present invention relates to a method of labeling a target-binding antibody to form an immuno-labeled complex suitable for use in the detection and measurement of one or more targets in a biological sample. The invention has applications in the fields of molecular biology, cell biology, immunohistochemistry, diagnostics, and therapeutics.

### Background of the Invention

Immunolabeling is a method for qualitative or quantitative determination of the presence of a target in a sample, wherein antibodies are utilized for their specific binding capacity. The antibodies form a complex with the target (antigen), wherein a detectable label is present on the antibody or on a secondary antibody. The detectable label is a key feature of immunolabeling, which can be detected directly or indirectly. The label provides a measurable signal by which the binding reaction is monitored providing a qualitative and/or quantitative measure of the degree of binding. The relative quantity and location of signal generated by the labeled antibodies can serve to indicate the location and/or concentration of the target. The label can also be used to select and isolate labeled targets, such as by flow sorting or using magnetic separation media. Examples of labels include but are not limited to radioactive nucleotides (¹²⁵I, ³H, ¹⁴C, ³²P), chemiluminescent, fluorescent, or phosphorescent compounds (e.g., dioxetanes, xanthene, or carbocyanine dyes, lanthanide chelates), particles (e.g., gold clusters, colloidal gold, microspheres, quantum dots), and enzymes (e.g., peroxidases, glycosidases, phosphatases, kinases). Ideally, the label is attached to the antibody in a manner that does not perturb the antibody's binding characteristics but enables the label to be measured by an appropriate detection technology. The choice of labels is influenced by factors such as ease and sensitivity of detection, equipment availability, background in the sample (including other labels) and the degree to which such labels are readily attached to the particular antibody. Both direct and indirect labeling of antibodies is utilized for immunolabeling. Direct labeling utilizes only a primary antibody, i.e. the antibody specific for the target, bound to the label. In contrast, indirect labeling utilizes a secondary antibody bound to the label, which is specific for the primary antibody, e.g. a goat anti-rabbit antibody. The principal differences in immunolabeling methods and materials reside in the way that the label is attached to the antibody-antigen complex, the type of label that is used, and the means by which the antibody-antigen complex is detected.

Limitations for direct labeling primary antibodies include the need for buffers free of primary amines, or carrier proteins such as bovine serum albumin (BSA), and other compounds such as tris-(hydroxymethyl)aminomethane (TRIS), glycine, and ammonium ions. These materials are, however, common components in antibody buffers and purification methods, and it may not be possible or feasible to remove them prior to the coupling reaction. In particular, many monoclonal antibodies are available only as ascites fluid or in hybridoma culture supernatants, or diluted with carrier proteins, such as albumins. Thus, direct labeling of antibodies in ascites fluid or other medias containing interfering compounds is not attainable.

The indirect immunolabeling method typically involves a multi-step process in which an unlabeled first antibody (typically a primary antibody) is directly added to the sample to form a complex with the antigen in the sample. Subsequently, a labeled secondary antibody, specific for the primary antibody, is added to the sample, where it attaches noncovalently to the primary antibody-antigen complex. Alternatively, a detectable label is covalently attached to an immunoglobulin-binding protein such as protein A and protein G to detect the antibody-antigen complex that has previously been formed with the target in the sample. Using ligands, such as streptavidin, that are meant to amplify the detectable signal also expands this cascade binding.

Indirect immunolabeling often results in false positives and high background. This is due to the fact that secondary antibodies, even when purified by adsorption against related species, nevertheless can exhibit significant residual cross-reactivity when used in the same sample. For example, when mouse tissue is probed with a mouse monoclonal antibody, the secondary antibody must necessarily be a labeled anti-mouse antibody. This anti-mouse antibody will detect the antibody of interest but will inevitably and additionally detect irrelevant, endogenous mouse immunoglobulins inherent in mouse tissue. This causes a significant background problem, especially in diseased tissues, which reduces the usefulness and sensitivity of the assay. Thus, the simultaneous detection of more than one primary antibody in a sample without this significant background Interference depends on the availability of secondary antibodies that 1) do not cross-react with proteins intrinsic to the sample being examined, 2) recognize only one of the primary antibodies, and 3) do not recognize each other (Brelje, et al., METHODS IN CELL BIOLOGY 38, 97-181, especially 111-118 (1993)).

To address the background problem in indirect labeling, a number of strategies have been developed to block access of the anti-mouse secondary antibodies to the endogenous mouse immunoglobulins. One such strategy for blocking involves complexing the primary antibody with a selected biotinylated secondary antibody to produce a complex of the primary and secondary antibodies, which is then mixed with diluted normal murine serum (Trojanowski et al., U.S. Pat No. 5,281,521 (1994)). This method is limited by the necessity to utilize an appropriate ratio of primary-secondary complex. Too low a ratio of primary-secondary complex will cause a decrease in specific staining and increased background levels due to the uncomplexed secondary anti-mouse antibody binding to endogenous mouse antibodies. However, the ability of a whole IgG antibody (as was used in the referenced method) to simultaneously bind and cross-link two antigens results in too high a ratio, causing the complex to precipitate or form complexes that are too large to penetrate into the cell or tissue.

Another strategy for blocking access to endogenous immunoglobulins in the sample involves pre-incubating the sample with a monovalent antibody, such as Fab' fragments, from an irrelevant species that recognize endogenous immunoglobulins. This approach requires large quantities of expensive Fab' fragments and gives mixed results and adds at least two steps (block and wash) to the overall staining procedure. The addition of a cross-linking reagent has resulted in improved reduction of background levels (Tsao, et al., U.S. Pat. No. 5,869,274 (1997)) but this is problematic when used with fluorophore-labeled antibodies. The cross-linking causes an increase in the levels of autofluorescence and thus the background (J. Neurosci. Meth. 83, 97 (1998); Mosiman et al., Methods 77, 191 (1997); Commun. Clin. Cytometry 30, 151 (1997); Beisker et al., Cytometry 8, 235 (1987)). In addition, pre-incubation with a cross-linking reagent often masks or prevents the antibody from binding to its antigen (J. Histochem. Cytochem. 45, 327 (1997); J. Histochem. Cytochem. 39, 741 (1991); J. Histochem. Cytochem. 43, 193 (1995); Appl. Immunohistochem. Molecul. Morphol. 9, 176 (2001)).

In a variation of this blocking strategy, a multi-step sequential-labeling procedure is used to overcome the problems of cross-reactivity. The sample is incubated with a first antibody to form a complex with the first antigen, followed by incubation of the sample with a fluorophore-labeled goat Fab anti-mouse IgG to label the first antibody and block it from subsequently complexing when the second antibody is added. In the third step, a second mouse antibody forms a complex with the second antigen. Because the second antibody is blocked from cross-reacting with the first antibody, the second mouse antibody is detected with a standard indirect-labeling method using a goat anti-mouse antibody conjugated to a different fluorescent dye ( J. Histochem. Cytochem. 34, 703 (1986)). This process requires multiple incubation steps and washing steps and it still cannot be used with mouse antibodies to probe mouse tissue.

Another blocking method is disclosed in the animal research kit (ARK) developed by DAKO, which is discussed in van der Loos et al., J. Histochem. & Cytochem. (2000) 48, 1431-1437. In this kit, a primary antibody is complexed with biotin-labeled goat Fab anti-mouse IgG and excess free Fab is blocked with normal mouse serum. However, since the Fab used in this process is generated from the intact IgG (rather than a selected region) there is a potential for the formation of anti-paratope or anti-idiotype antibodies that will block the antigen-binding site and prevent immunolabeling. The biotinylated antibody also requires subsequent addition of a labeled avidin or streptavidin conjugate for its subsequent visualization.

The present invention is advantageous over previously described methods for labeling a target-binding antibody in that it provides the benefits of indirect labeling with the ease and flexibility of direct labelling. The present invention employs labeled monovalent antibody fragments (Fab or Fab¹ fragments) specific for the Fc region of a target-binding antibody, which are complexed prior to addition with a biological sample. Because these monovalent proteins are not bivalent antibodies, precipitation and cross-linking are not a problem. Therefore the methods of the present invention can be used with immunologically similar monoclonal or polyclonal antibodies of either an identical isotype or different isotypes. As the monovalent labeling reagents are specific for the Fc region of target-binding antibodies, these reagents will not interfere with the binding region of the primary antibody. In addition, the monovalent labeling proteins are not negatively affected by the presence of primary amines like BSA, gelatin, hybridoma culture supernatants or ascites fluid and thus primary antibodies present in these media can be effectively labeled with such labeling reagents. Thus, the present invention provides numerous advantages over the conventional methods of immunolabeling.

### SUMMARY OF THE INVENTION

The present invention provides a method of labeling a target-binding antibody, wherein said method comprises the steps of:
a) contacting a solution of target-binding antibodies with a labeling reagent subset, wherein said labeling reagent subsets are distinguished by i) ratio of label to labeling reagent or ii) a physical properties of said label;
b) incubating said target-binding antibodies and said labeling reagent subset for a time period sufficient for one or more labeling reagents to form an immuno-labeled complex with a target-binding antibody wherein a region of said target binding antibody is selectively bound by labeling reagent;
c) optionally removing unbound labeling reagent by adding a capture reagent comprising immunoglobulin proteins or fragments thereof; and,
d) optionally repeating said steps a), b), and c) to form a panel of immuno-labeled complex subsets wherein each subset is distinguished from another subset by i) a ratio of label to labeling reagent, or ii) a physical property of said label, or iii) a ratio of labeling reagent to said target-binding antibody, or iv) by said target-binding antibody, wherein said labelling reagent is an anti-Fc region Fab or Fab' fragment.Typically, the labeling reagent is an anti-Fc Fab or Fab' fragment that was generated by immunizing a goat or rabbit with the Fc fragment of an antibody.

If optional step c) is carried out, the capture reagent comprising immunoglobulin proteins or fragments thereof may be immobilized on a matrix. The labeling of the target-binding antibody can be performed irrespective of the solution that the antibody is present in and includes proteins that are normally present in serum or ascites. This feature of the labeling process of the target-binding antibody eliminates the need to purify and concentrate the target-binding antibody. The time required for the labeling reagent to selectively bind to the target-binding antibody is typically very short, often less than 10 minutes. Often the labeling reagent binds the target-binding antibody in the amount of time it takes to add and mix the labeling reagent with the target-binding antibody.

As indicated above, the labeling steps of the target-binding antibody are optionally repeated to form a panel of subsets. These immuno-labeled complex subsets may be used individually or pooled wherein each subset is distinguished from another subset by i) the target-binding antibody, or ii) a ratio of label to labeling reagent, or iii) a ratio of labeling reagent to the target-binding antibody or iv) by a physical property of the label. Thus, it is appreciated that a wide range of subsets can be formed wherein the subsets can be used individually to detect a target in a sample or pooled to simultaneously detect multiple targets in a sample. The simultaneous detection of multiple targets in a sample is especially useful in methods that utilize flow cytometry or methods that immobilize a population of cells or tissue on a surface.

The target binding antibody may be a murine monoclonal antibody, a rabbit polyclonal antibody or a goat polyclonal antibody. As indicated above, the target-binding antibodies may be in a solution comprising serum proteins or ascites proteins.

Suitable labels may be selected from the group consisting of a chromophore, a fluorophore (e.g. coumarin, a xanthene, a cyanine, a pyrene, a borapolyazaindacene, an oxazine, or a derivative thereof), a fluorescent protein such as a phycobiliprotein, a phosphorescent dye, a tandem dye (e.g. a cyanine-phycobiliprotein derivative or xanthenes-phycobiliprotein derivative), a particle (e.g. a microsphere or a quantum dot), a hapten, an enzyme (e.g. selected from the group consisting of a peroxidise, a phosphatase, a glycosidase, and a luciferase) and a radioisotope.

### BRIEF DESCRIPTION OF THE DRAWINGS

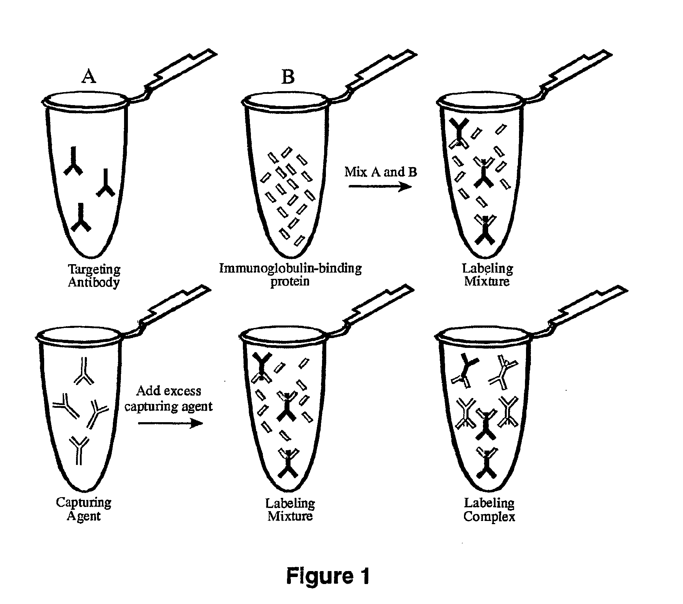

**Figure 1**: Shows a schematic representation of the formation of the immuno-labeled complex (target-binding antibody and labeling reagent).

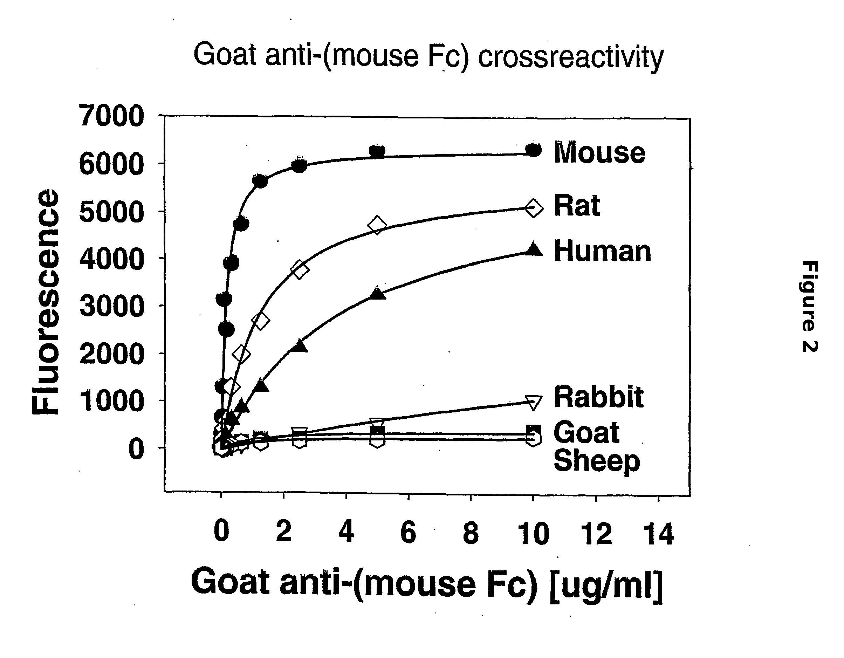

**Figure 2**: Shows species specificity of goat Fab anti-(mouse Fc), as observed using a microplate coated with IgG of various species. The various species were blocked with BSA, reacted with biotinylated goat Fab anti-(mouse Fc), washed, and then treated with streptavidin-horseradish peroxidase (HRP), followed by hydrogen peroxide (H₂O₂) and the Amplex Red peroxidase detection reagent.

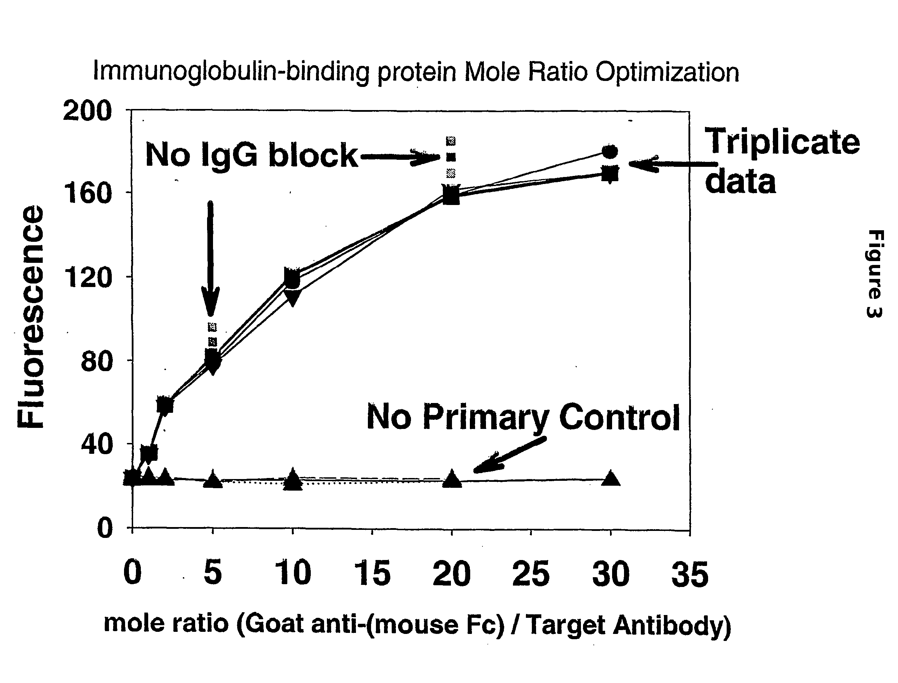

**Figure 3**: Shows a preferred molar ratio of a goat Fab anti-(mouse Fc) labeling reagent. Varying amounts of an Alexa Fluor 488 dye-labeled Fab fragment of goat anti-(mouse Fc) were added to a constant amount of anti-biotin monoclonal antibody (mAb). This mixture was equilibrated for 20 minutes, and then added to biotinylated-BSA in a microplate well. After allowing time to bind, the plates were washed and the remaining fluorescence was quantitated. The analysis was performed in triplicate (circles). Control experiments were performed, as described above, but without adding the primary anti-biotin antibody (solid squares).

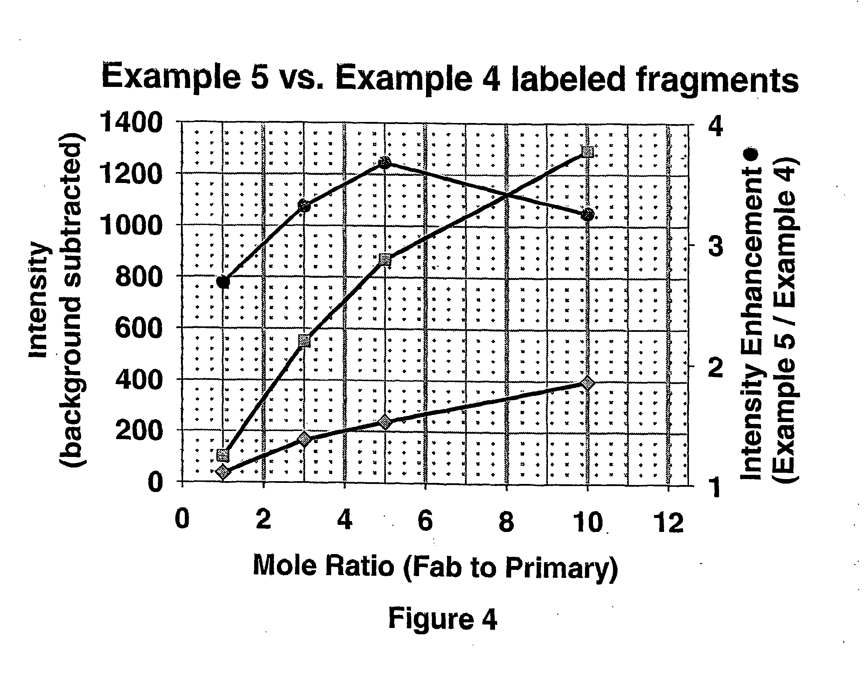

**Figure 4**: Shows a comparison of the fluorescence intensity (Example 6) for labeling reagent prepared in homogeneous solution (Example 4) and labeling reagent prepared on a column (Example 5).

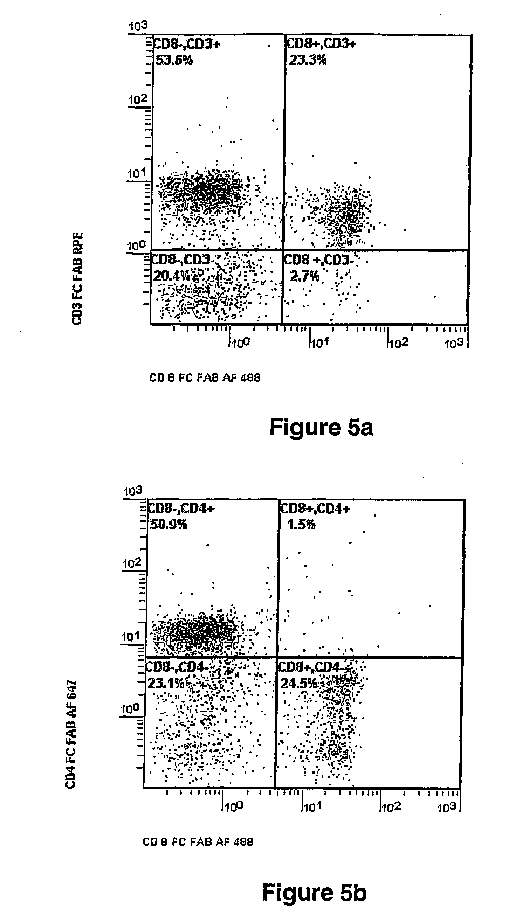

**Figure 5****:** Shows detection of multiple targets on T cells using a labeling reagent attached to a R-phycoerythrin (R-PE) (Fig. 5A) to detect CD3-positive T cells, a labeling reagent attached to Alexa Fluor 647 dye (Fig: 5B) to detect CD4-positive T cells and a labeling reagent attached to Alexa Fluor 488 dye (Fig 5B) to detect CD8-positive T cells (Example 18). The CD-3 detected T cells are shown in the upper left (UL) and upper right (UR) quadrants. The relative percentages of total lymphocytes that are CD3-positive cells are 83.3% (UL+UR). The relative percentage of CD8-positive Alexa Fluor 488 dye-stained lymphocytes and CD3-positive R-PE dye-stained lymphocytes is 35.1% (UR quadrant). The lower left quadrant (LL, 20.4%) shows CD3-negative lymphocytes (i.e. non-T cells) comprised of NK cells, B cells and some monocytes. In the lower right (LR, 2.7%) region are non-T cells, which are nonspecifically stained. Figure 5B further shows CD3-positive T-cells subdivided into Alexa Fluor 647 dye CD4-positive and Alexa Fluor 488 dye CD8-positive. CD4-positive cells represent 50.9% of total lymphocytes (UL quadrant) and CD8-positive cells represent 24.5% of the total lymphocytes (LR quadrant). The 23.1 % of cells in the L.L quadrant are non-T cells, while the 1.5% of cells in UR quadrant are likely non-specifically stained lymphocytes.

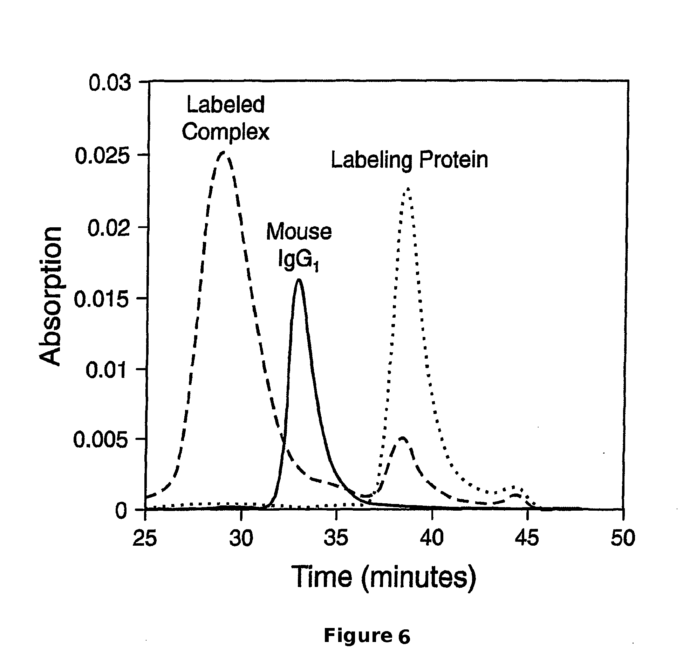

**Figure 6****:** Shows high-performance size-exclusion chromatographic analysis of Alexa Fluor 488 dye-labeled goat Fab anti-(mouse Fc) labeling reagent binding to a mouse IgG1 target-binding antibody. The labeling reagent, alone, appears as a peak at 38 minutes; the target-binding antibody, alone, appears as a peak at 33 minutes. When labeling reagent and target-binding antibody are mixed together at a molar ratio of 5:1 (labeling reagent:target-binding antibody), the resulting immunolabeling complex appears as a peak at 29 minutes (**Example 10**).

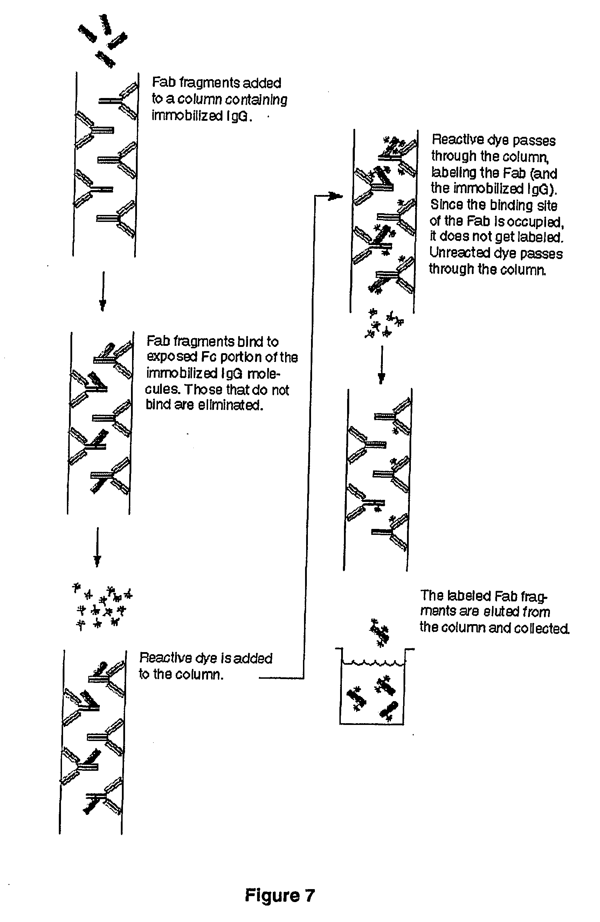

**Figure 7****:** Shows the production of labeling reagent wherein the label is attached to the labeling reagent when immobilized on a column.

### DETAILED DESCRIPTION OF THE INVENTION

### I. DEFINITIONS

It should be noted that, as used in this specification and the appended claims, the singular form "a", "an" and "the" include plural referents unless the context clearly dictates otherwise. Thus, for example, reference to "a protein labeling complex" includes a plurality of complexes and reference to "a target-binding protein" includes a plurality of proteins and the like.

Unless defined otherwise, all technical and scientific terms used herein have the same meaning as commonly understood by one of ordinary skill in the art to which this invention is related. The following terms are defined for purposes of the invention as described herein.

The term "affinity" as used herein refers to the strength of the binding interaction of two molecules, such as an antibody and an antigen or a positively charged moiety and a negatively charged moiety. For bivalent molecules such as antibodies, affinity is typically defined as the binding strength of one binding domain for the antigen, e.g. one Fab fragment for the antigen. The binding strength of both binding domains together for the antigen is referred to as "avidity". As used herein "High affinity" refers to a ligand that binds to an antibody having an affinity constant (Kₐ) greater than 10⁴ M⁻¹, typically 10⁵-10¹¹ M⁻¹; as determined by inhibition ELISA or an equivalent affinity determined by comparable techniques such as, for example, Scatchard plots or using K_{d}/dissociation constant, which is the reciprocal of the Kₐ, etc.

The term "antibody" as used herein refers to a protein of the immunoglobulin (Ig) superfamily that binds noncovalently to certain substances (e.g. antigens and immunogens) to form an antibody-antigen complex. Antibodies can be endogenous, or polyclonal wherein an animal is immunized to elicit a polyclonal antibody response or by recombinant methods resulting in monoclonal antibodies produced from hybridoma cells or other cell lines. It is understood that the term "antibody" as used herein includes within its scope any of the various classes or sub-classes of immunoglobulin derived from any of the animals conventionally used.

The term "antibody fragments" as used herein refers to fragments of antibodies that retain the principal selective binding characteristics of the whole antibody. Particular fragments are well-known in the art, for example, Fab, Fab', and F(ab')₂, which are obtained by digestion with various proteases, pepsin or papain, and which lack the Fc fragment of an intact antibody or the so-called "half-molecule" fragments obtained by reductive cleavage of the disulfide bonds connecting the heavy chain components in the intact antibody. Such fragments also include isolated fragments consisting of the light-chain-variable region, "Fv" fragments consisting of the variable regions of the heavy and light chains, and recombinant single chain polypeptide molecules in which light and heavy variable regions are connected by a peptide linker. Other examples of binding fragments include (i) the Fd fragment, consisting of the VH and CH1 domains; (ii) the dAb fragment ( Ward, et al., Nature 341,544 (1989)), which consists of a VH domain; (iii) isolated CDR regions; and (iv) single-chain Fv molecules (scFv) described above. In addition, arbitrary fragments can be made using recombinant technology that retains antigen-recognition characteristics.

The term "antigen" as used herein refers to a molecule that induces, or is capable of inducing, the formation of an antibody or to which an antibody binds selectively, including but not limited to a biological material. Antigen also refers to "immunogen". The target-binding antibodies selectively bind an antigen, as such the term can be used herein interchangeably with the term "target""

The term "biotin" as used herein refers to any biotin derivative, including without limitation, substituted and unsubstituted biotin, and analogs and derivatives thereof, as well as substituted and unsubstituted derivatives of caproylamidobiotin, biocytin, desthiobiotin, desthiobiocytin, iminobiotin, and biotin sulfone.

The term "biotin-binding protein" as used herein refers to any protein that binds selectively and with high affinity to biotin, including without limitation, substituted or unsubstituted avidin, and analogs and derivatives thereof, as well as substituted and unsubstituted derivatives of streptavidin, ferritin avidin, nitroavidin, nitrostreptavidin, and Neutravidin^{™} avidin (a deglycosylated modified avidin having an isoelectric point near neutral).

The term "buffer" as used herein refers to a system that acts to minimize the change in acidity or basicity of the solution against addition or depletion of chemical substances.

The term "capture reagent" refers to a non-specific immunoglobulin that is used to remove excess labeling reagent after the formation of the immuno-labeled complex. The capture reagent is optionally attached a matrix to facilitate removal of the excess labeling regent. A matrix typically includes a microsphere, an agarose bead or any solid surface that the excess labeling reagent can be passed by.

The term "chromophore" as used herein refers to a label that emits light in the visible spectra that can be observed without the aid of instrumentation.

The term "complex" as used herein refers to the association of two or more molecules, usually by non-covalent bonding, e.g., the association between an antibody and an antigen or the labeling reagent and the target-binding antibody.

The term "detectable response" as used herein refers to an occurrence of, or a change in, a signal that is directly or indirectly detectable either by observation or by instrumentation. Typically, the detectable response is an occurrence of a signal wherein the fluorophore is inherently fluorescent and does not produce a change in signal upon binding to a metal ion or biological compound. Alternatively, the detectable response is an optical response resulting in a change in the wavelength distribution patterns or intensity of absorbance or fluorescence or a change in light scatter, fluorescence lifetime, fluorescence polarization, or a combination of the above parameters. Other detectable responses include, for example, chemiluminescence, phosphorescence, radiation from radioisotopes, magnetic attraction, and electron density.

The term "detectably distinct" as used herein refers to a signal that is distinguishable or separable by a physical property either by observation or by instrumentation. For example, a fluorophore is readily distinguishable either by spectral characteristics or by fluorescence intensity, lifetime, polarization or photo-bleaching rate from another fluorophore in the sample, as well as from additional materials that are optionally present.

The term "directly detectable" as used herein refers to the presence of a material or the signal generated from the material is immediately detectable by observation, instrumentation, or film without requiring chemical modifications or additional substances.

The term "examination zone" as used herein refers to an optical zone of a flow cytometer, or a similar instrument, wherein cells are passed through essentially one at a time in a thin stream whereby the bound immuno-labeled complex is illuminated and the intensity and emission spectra of the fluorophore is detected and recorded. This includes instruments wherein the examination zone moves and the sample is held in place.

The term "fluorophore" as used herein refers to a composition that is inherently fluorescent or demonstrates a change in fluorescence upon binding to a biological compound or metal ion, i.e., fluorogenic. Fluorophores may contain substitutents that alter the solubility, spectral properties or physical properties of the fluorophore. Numerous fluorophores are known to those skilled in the art and include, but are not limited to coumarin, cyanine, benzofuran, a quinoline, a quinazolinone, an indole, a benzazole, a borapolyazaindacene and xanthenes including fluoroscein, rhodamine and rhodol as well as other fluorophores described in RICHARD P. HAUGLAND, MOLECULAR PROBES HANDBOOK OF FLUORESCENT PROBES AND RESEARCH CHEMICALS (9th edition, CD-ROM, September 2002).

The term "immuno-labeled complex" refers to the complex of target-binding antibody that is non-covalently attached to a labeling reagent as defined in the claims.

The term "immuno-labeled complex subset" as used herein refers to a discrete set of immuno-labeled complexes that are homogenous and can be distinguished from another subset of immuno-labeled complex by the physical properties of the label, or the ratio of the label to labeling reagent, or the ratio of labeling reagent to target-binding antibody, or the target-binding antibody.

The term "label" as used herein refers to a chemical moiety or protein that retains its native properties (e.g. spectral properties, conformation and activity) when attached to a labeling reagent and used in the present methods. The label can be directly detectable (fluorophores) or indirectly detectable (hapten or enzyme). Such labels include, but are not limited to, radiolabels that can be measured with radiation-counting devices; pigments, dyes or other chromogens that can be visually observed or measured with a spectrophotometer; spin labels that can be measured with a spin label analyzer; and fluorescent labels (fluorophores), where the output signal is generated by the excitation of a suitable molecular adduct and that can be visualized by excitation with light that is absorbed by the dye or can be measured with standard fluorometers or imaging systems, for example. The label can be a chemiluminescent substance, where the output signal is generated by chemical modification of the signal compound; a metal-containing substance; or an enzyme, where there occurs an enzyme-dependent secondary generation of signal, such as the formation of a colored product from a colorless substrate. The term label can also refer to a "tag" or hapten that can bind selectively to a conjugated molecule such that the conjugated molecule, when added subsequently along with a substrate, is used to generate a detectable signal. For example, one can use biotin as a tag and then use an avidin or streptavidin conjugate of horseradish peroxidate (HRP) to bind to the tag, and then use a colorimetric substrate (e.g., tetramethylbenzidine (TMB)) or a fluorogenic substrate such as Amplex Red reagent (Molecular Probe, Inc.) to detect the presence of HRP. Numerous labels are know by those of skill in the art and include, but are not limited to, particles, fluorophore, haptens, enzymes and their colorimetric, fluorogenic and chemiluminescent substrates and other labels that are described in RICHARD P. HAUGI-AND, MOLECULAR PROBES HANDBOOK OF FLUORESCENT PROBES AND RESEARCH PRODUCTS (9th edition, CD-ROM, September 2002 ), supra.

The term "labeling reagent" as used herein refers to a monovalent antibody fragment which is a Fab or Fab' fragment provided that the labeling reagent has affinity for the Fc region of the target-binding antibody and is covalently attached to a label. The label covalently attached to a labeling reagent is directly detectable such as a fluorophore or functions as an indirect label that requires an additional component such as a colorimetric enzyme substrate or an enzyme conjugate.

The term "labeling reagent subset" as used herein refers to a discrete set of labeling reagents that are homogenous and can be distinguished from another subset of labeling reagent either by the physical properties of the label or the ratio of the label to labeling reagent.

The term "labeling solution" as used herein refers to a solution that is used to form an immuno-labeled complex wherein the solution comprises labeling reagents and a buffer.

The term "matrix" as used herein refers to a solid or semi-solid surface that a biological molecule can be attached to, such as a sample of the present invention or a capture reagent. Examples include, but are not limited to, agarose, polyacrylamide gel, polymers, microspheres, glass surface, plastic surface, membrane, margnetic surface, and an array.

The term "monovalent antibody fragment" as used herein refers to an antibody fragment that has only one antigen-binding site. Examples of monovalent antibody fragments include, but are not limited to, Fab fragments (no hinge region), Fab' fragments (monovalent fragments that contain a heavy chain hinge region), and single-chain fragment variable (ScFv) proteins. The terms "protein" and "polypeptide" are used herein in a generic sense to include polymers of amino acid residues of any length. The term "peptide" is used herein to refer to polypeptides having less than 100 amino acid residues, typically less than 10 amino acid residues. The terms apply to amino acid polymers in which one or more amino acid residues are an artificial chemical analogue of a corresponding naturally occurring amino acid, as well as to naturally occurring amino acid polymers.

The term "purified" as used herein refers to a preparation of a target-binding antibody that is essentially free from contaminating proteins that normally would be present in association with the antibody, e.g., in a cellular mixture or milieu in which the protein or complex is found endogenously such as serum proteins or hybridoma supernatant.

The term "sample" as used herein refers to any material that may contain a target, as defined below. Typically, the sample comprises a population of cells, cellular extract, subcellular components, tissue culture, a bodily fluid, and tissue. The sample may be in an aqueous solution, a viable cell culture or immobilized on a solid or semi solid surface such as a gel, a membrane, a glass surface, a microparticle or on a microarray.

The term "target" as used herein refers to any entity that a target-binding antibody has affinity for such as an epitope or antigen. This target includes not only the discrete epitope that the target-binding antibody has affinity for but also includes any subsequently bound molecules or structures. In this way an epitope serves as a marker for the intended target. For example, a cell is a target wherein the target-binding antibody binds a cell surface protein such as CD3 on a T cell wherein the target marker is CD3 and the target is the T cell.

The term "target-binding antibody" as used herein refers to an antibody that has affinity for a discrete epitope or antigen that can be used with the methods described herein. Typically the discrete epitope is the target but the epitope can be a marker for the target such as CD3 on T cells. The term can be used interchangeably with the term "primary antibody" when describing methods that use an antibody that binds directly to the antigen as opposed to a "secondary antibody" that binds to a region of the primary antibody.

The target-binding antibodies are labeled with the labeling reagent in a labeling method to form immuno-labeled complexes and then added to a sample to detect a target.

### A. Labeling Reagents

As indicated above, the labeling reagents employed in a method of the present invention are monovalent antibody fragments that have affinity for the Fc region of a target-binding antibody.

The Fc region is preferable because it is the farthest from the binding domain of the target-binding antibody. When bound by a labeling reagent, steric hinderance of the binding domain for the target is unlikely to be caused.

Antibody is a term of the art denoting the soluble substance or molecule secreted or produced by an animal in response to an antigen, and which has the particular property of combining specifically with the antigen that induced its formation. Antibodies themselves also serve are antigens or immunogens because they are glycoproteins and therefore are used to generate anti-species antibodies. Antibodies, also known as immunoglobulins, are classified into five distinct classes-IgG, IgA, IgM, IgD, and IgE. The basic IgG immunoglobulin structure consists of two identical light polypeptide chains and two identical heavy polypeptide chains (linked together by disulfide bonds). When IgG is treated with the enzyme papain, a monovalent antigen-binding fragment can be isolated, referred herein to as a Fab fragment. When IgG is treated with pepsin (another proteolytic enzyme), a larger fragment is produced, F(ab')2. This fragment can be split in half by treating with a mild reducing buffer that results in the monovalent Fab' fragment. The Fab' fragment is slightly larger than the Fab and contains one or more free sulfhydryls from the hinge region (which are not found in the smaller Fab fragment). The term "antibody fragment" is used herein to define both the Fab' and Fab portions of the antibody. It is well known in the art to treat antibody molecules with pepsin and papain in order to produce antibody fragments (Gorevic et al., Methods of Enzyol., 116:3 (1985)).

The monovalent Fab fragments used in the present invention are produced from either murine monoclonal antibodies or polyclonal antibodies generated in a variety of animals that have been immunized with a foreign antibody or fragment thereof, US Patent No. 4,196,265 discloses a method of producing monoclonal antibodies. Typically, labeling reagents are derived from a polyclonal antibody that has been produced in a rabbit or goat but any animal known to one skilled in the art to produce polyclonal antibodies can be used to generate anti-species antibodies. However, monoclonal antibodies are equal, and in some cases, preferred over polyclonal antibodies provided that the target-binding antibody is compatible with the monoclonal antibodies that are typically produced from murine hybridoma cell lines using methods well known to one skilled in the art. Example 1 describes production of polyclonal antibodies raised in animals immunized with the Fc region of a foreign antibody. The labeling reagents for use in the present invention are generated against only the Fc region of a foreign antibody. Essentially, the animal is immunized with only the Fc region fragment of a foreign antibody, such as murine. The polyclonal antibodies are collected from subsequent bleeds, digested with an enzyme, pepsin or papain, to produce monovalent fragments. The fragments are then affinity purified on a column comprising whole immunoglobulin protein that the animal was immunized against or just the Fc fragments. As described in detail below, the labeling reagents are also covalently labeled with fluorophore labels when bound to the affinity column to eliminate incorporating label into the binding domain of the monovalent fragment. One of skill in the art will appreciate that this method can be used to generate monovalent fragments against any region of a target-binding protein and that selected peptide fragments of the target-binding antibody could also be used to generate fragments.

### 2. Labels

Labels for use in a method of the invention include any directly or indirectly detectable label known by one skilled in the art that can be covalently attached to the labeling reagent. Labels include, without limitation, a chromophore, a fluorophore, a fluorescent protein, a phosphorescent dye, a tandem dye, a particle, a hapten, an enzyme and a radioisotope. Preferred labels include fluorophores, fluorescent proteins, haptens, and enzymes.

A fluorophore of the present invention is any chemical moiety that exhibits an absorption maximum beyond 280 nm, and when covalently attached to a labeling reagent retains its spectral properties. Fluorophores useful in the present invention include, without limitation; a pyrene (including any of the corresponding derivative compounds disclosed in US Patent 5,132,432), an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a cyanine (including any corresponding compounds in US Serial Nos. 09/968,401 and 09/969,853 , a carbocyanine (including any corresponding compounds in US Serial No. 09/557;275 ; U.S.; Patents Nos. 4,981,977; 5,268,486; 5,569,587; 5,569,766; 5,486,616; 5,627,027; 5,808,044; 5,877,310; 6,002,003; 6,004,536; 6,008,373; 6,043,025; 6,127,134; 6,130,094; 6,133,445; and publications WO 97/40104, WO 99/51702 , WO 01/21624 ; EP 1 065 250 A1), a carbostyryl, a porphyrin, a salicylate, an anthranilate, an azulene, a perylene, a pyridine, a quinoline, a borapolyazaindacene (including any corresponding compounds disclosed in US Patent Nos. 4,774,339 ; 5,187,288 ; 5,248,782 ; 5,274,113 ; and 5,433,896 ), a xanthene (including any corresponding compounds disclosed in U.S. Patent No. 6,162,931; 6,130,101; 6,229,055; 6,339,392; 5,451,343 and US serial No. 09/922,333, an oxazine (including any corresponding compounds disclosed in US Patent No. 4,714,763) or a benzoxazine, a carbazine (including any corresponding compounds disclosed in US Patent No. 4,810,888), a phenalenone, a coumarin (including an corresponding compounds disclosed in US Patent Nos. 5,696,157; 5,459,276; 5,501,980 and 5,830,912 ), a benzofuran (including an corresponding compounds disclosed in US Patent Nos. 4,603,209 and 4,849,362) and benzphenalenone (including any corresponding compounds disclosed in US Patent No. 4,812,409) and derivatives thereof. As used herein, oxazines include resorufins (including any corresponding compounds disclosed in 5,242,805), aminooxazinones, diaminooxazines, and their benzo-substituted analogs.

When the fluorophore is a xanthene, the fluorophore is optionally a fluorescein, a model (including any corresponding compounds disclosed in US Patent Nos. 5,227,487 and 5,442,045 ), or a rhodamine (including any corresponding compounds in US Patent Nos. 5,798,276 ; 5,846,737 ; US serial no. 09/129,015. As used herein, fluorescein includes benzo- or dibenzofluoresceins, seminaphthofluoresceins, or naphthofluoresceins. Similarly, as used herein rhodol includes seminaphthorhodafluors (including any corresponding compounds disclosed in U.S. Patent No. 4,945,171). Alternatively, the fluorophore is a xanthene that is bound via a linkage that is a single covalent bond at the 9-position of the xanthene. Preferred xanthenes include derivatives of 3H-xanthen-6-ol-3-one attached at the 9-position, derivatives of 6-amino-3H-xanthen-3-one attached at the 9-position, or derivatives of 6-amino-3H-xanthen-3-imine attached at the 9-position.

Preferred fluorophores for use in the invention include xanthene (rhodol, rhodamine, fluorescein and derivatives thereof) coumarin, cyanine, pyrene, oxazine and borapolyazaindacene. Most preferred are sulfonated xanthenes, fluorinated xanthenes, sulfonated coumarins, fluorinated coumarins and sulfonated cyanines. The choice of the fluorophore attached to the labeling reagent will determine the absorption and fluorescence emission properties of the labeling reagent and immuno-labeled complex. Physical properties of a fluorophore label include spectral characteristics (absorption, emission and stokes shift), fluorescence intensity, lifetime, polarization and photo-bleaching rate all of which can be used to distinguish one fluorophore from another.

Typically the fluorophore contains one or more aromatic or heteroaromatic rings, that are optionally substituted one or more times by.a variety of substituents, including without limitation, halogen, nitro, cyano, alkyl, perfluoroalkyl, alkoxy, alkenyl, alkynyl, cycloalkyl, arylalkyl, acyl, aryl or heteroaryl ring system, benzo, or other substituents typically present on fluorophores known in the art.

The fluorophore may have an absorption maximum beyond 480 nm, e.g. the fluorophore absorbs at or near 488 nm to 514 nm (particularly suitable for excitation by the output of the argon-ion laser excitation source) or near 546 nm (particularly suitable for excitation by a mercury arc lamp).

Many of fluorophores can also function as chromophores and thus the described fluorophores are also preferred chromophores for use in the present invention.

In addition to fluorophores, enzymes also find use as labels for the labeling reagents. Enzymes are desirable labels because amplification of the detectable signal can be obtained resulting in increased assay sensitivity. The enzyme itself does not produce a detectable response but functions to break down a substrate when it is contacted by an appropriate substrate such that the converted substrate produces a fluorescent, colorimetric or luminescent signal. Enzymes amplify the detectable signal because one enzyme on a labeling reagent can result in multiple substrates being converted to a detectable signal. This is advantageous where there is a low quantity of target present in the sample or a fluorophore does not exist that will give comparable or stronger signal than the enzyme. However, fluorophores are most preferred because they do not require additional assay steps and thus reduce the overall time required to complete an assay. The enzyme substrate is selected to yield the preferred measurable product, e.g. colorimetric, fluorescent or chemiluminescence. Such substrates are extensively used in the art, many of which are described in the MOLECULAR PROBES HANDBOOK, *supra.*

A preferred colorimetric or fluorogenic substrate and enzyme combination uses oxidoreductases such as horseradish peroxidase and a substrate such as 3,3'-diaminobenzidine (DAB) and 3-amino-9-ethylcarbazole (AEC), which yield a distinguishing color (brown and red, respectively). Other colorimetric oxidoreductase substrates that yield detectable products include, but are not limited to: 2,2-azino-bis(3-ethylbenzothiazoline-6-sulfonic acid) (ABTS), o-phenylenediamine (OPD), 3,3',5,5'-tetramethylbenzidine (TMB), o-dianisidine, 5-aminosalicylic acid, 4-chloro-1-naphthol. Fluorogenic substrates include, but are not limited to, homovanillic acid or 4-hydroxy-3-methoxyphenylacetic acid, reduced phenoxazines and reduced benzothiazines, including Amplex^{®} Red reagent and its variants (U.S. Pat. No. 4,384,042) and reduced dihydroxanthenes, including dihydrofluoresceins (U.S. Pat. No. 6,162,931) and dihydrorhodamines including dihydrorhodamine 123. Peroxidase substrates that are tyramides (U.S. Pat. Nos. 5,196,306; 5,583,001 and 5,731,158) represent a unique class of peroxidase substrates in that they can be intrinsically detectable before action of the enzyme but are "fixed in place" by the action of a peroxidase in the process described as tyramide signal amplification (TSA). These substrates are extensively utilized to label targets in samples that are cells, tissues or arrays for their subsequent detection by microscopy, flow cytometry, optical scanning and fluorometry.

Another preferred colorimetric (and in some cases fluorogenic) substrate and enzyme combination uses a phosphatase enzyme such as an acid phosphatase, an alkaline phosphatase or a recombinant version of such a phosphatase in combination with a colorimetric substrate such as 5-bromo-6-chloro-3-indolyl phosphate (BCIP), 6-chloro-3-indolyl phosphate, 5-bromo-6-chloro-3-indolyl phosphate, *p*-nitrophenyl phosphate, or o-nitrophenyl phosphate or with a fluorogenic substrate such as 4-methylumbelliferyl phosphate, 6,8-difluoro-7-hydroxy-4-methylcoumarinyl phosphate (DIFMUP, U.S. Pat. No. 5,830,912) fluorescein diphosphate, 3-O-methylfluorescein phosphate, resorufin phosphate, 9*H*-(1,3-dichloro-9,9-dimethylacridin-2-one-7-yl) phosphate (DDAO phosphate), or ELF 97, ELF 39 or related phosphates (U.S. Pat. Nos. 5,316,906 and 5,443,986).

Glycosidases, in particular beta-galactosidase, beta-glucuronidase and beta-glucosidase, are additional suitable enzymes. Appropriate colorimetric substrates include, but are not limited to, 5-bromo-4-chloro-3-indolyl beta-D-galactopyranoside (X-gal) and similar indolyl galactosides, glucosides, and glucuronides, *o*-nitrophenyl beta-D-galactopyranoside (ONPG) and p-nitrophenyl beta-D-galactopyranoside. Preferred fluorogenic substrates include resorufin beta-D-galactopyranoside, fluorescein digalactoside (FDG), fluorescein diglucuronide and their structural variants (U.S. Pat. Nos. 5,208,148; 5,242,805; 5,362,628; 5,576,424 and 5,773,236), 4-methylumbelliferyl beta-D-galactopyranoside, carboxyumbelliferyl beta-D-galactopyranoside and fluorinated coumarin beta-D-galactopyranosides (U.S. Pat. No. 5,830,912).

Additional enzymes include; but are not limited to, hydrolases such as cholinesterases and peptidases, oxidases such as glucose oxidase and cytochrome oxidases, and reductases for which suitable substrates are known.

Enzymes and their appropriate substrates that produce chemiluminescence are preferred for some assays. These include, but are not limited to, natural and recombinant forms of luciferases and aequorins. Chemiluminescence-producing substrates for phosphatases, glycosidases and oxidases such as those containing stable dioxetanes, luminol, isoluminol and acridinium esters are additionally useful.

In addition to enzymes, haptens such as biotin are also preferred labels. Biotin is useful because it can function in an enzyme system to further amplify the detectable signal, and it can function as a tag to be used in affinity chromatography for isolation purposes. For detection purposes, an enzyme conjugate that has affinity for biotin is used, such as avidin-HRP. Subsequently a peroxidase substrate is added to produce a detectable signal.

Haptens also include hormones, naturally occurring and synthetic drugs, pollutants, allergens, affector molecules, growth factors, chemokines, cytokines, lymphokines, amino acids, peptides, chemical intermediates, nucleotides and the like.

Fluorescent proteins also find use as labels for the labeling reagents used in the present invention. Examples of fluorescent proteins include green fluorescent protein (GFP) and the phycobiliproteins and the derivatives thereof. The fluorescent proteins, especially phycobiliprotein, are particularly useful for creating tandem dye labeled labeling reagents. These tandem dyes comprise a fluorescent protein and a fluorophore for the purposes of obtaining a larger stokes shift wherein the emission spectra is farther shifted from the wavelength of the fluorescent protein's absorption spectra. This is particularly advantageous for detecting a low quantity of a target in a sample wherein the emitted fluorescent light is maximally optimized, in other words little to none of the emitted light is reabsorbed by the fluorescent protein. For this to work, the fluorescent protein and fluorophore function as an energy transfer pair wherein the fluorescent protein emits at the wavelength that the fluorophore absorbs at and the fluorphore then emits at a wavelength farther from the fluorescent proteins than could have been obtained with only the fluorescent protein. A particularly useful combination is the phycobiliproteins disclosed in US Patents 4,520,110; 4,859,582 ; 5,055,556 and the sulforhodamine fluorophores disclosed in 5,798,276 , or the sulfonated cyanine fluorophores disclosed in US serial Nos. 09/968/401 and 09/969/853; or the sulfonated xanthene derivatives disclosed in 6,130,101 and those combinations disclosed in US Patent 4,542,104 . Alternatively, the fluorophore functions as the energy donor and the fluorescent protein is the energy acceptor

### 3. Covalent Attachment of Labels to the Labeling Reagents

The labeling reagents can be independently attached to one or more labels by a number of methods known to one skilled in the art and modification of such methods. Methods include, labeling in a solution or on an affinity column. For labeling in solution the labeling reagent is optionally modified to contain a reactive group and the label is modified to contain a reactive group or is synthesized to contain a reactive group, as is typically the case with fluorophore labels wherein the reactive group facilitates covalent attachment. The modification of the labeling reagent to contain a reactive group includes (1) chemical addition of such a reactive group or (2) alternatively takes advantage of the disulfide bonds of the F(ab')₂ fragment wherein the fragment is reduced to break the bond and expose the thiol group that readily reacts with a reactive group on a label, as disclosed in US Patent No. 5,360,895. Typically, covalent attachment of the label to the fragment is the result of a chemical reaction between an electrophilic group and a nucleophilic group. However, when a label is used that is photoactivated the covalent attachment results when the labeling solution is illuminated.

A method for covalently attaching a label, particularly an enzyme, a fluorescent protein or a particle, comprises the following steps:
a) cleaving an intact anti-Fc antibody with an enzyme resulting in a F(ab')₂ fragment;
b) contacting said F(ab')₂ fragment with a reducing agent to produce Fab' fragments containing a thiol group;
c) contacting said Fab' fragments with a solution comprising a label that contains a reactive group; and,
d) isolating Fab' fragments of step d) that are covalently attached to a label by size exclusion or affinity chromatography.

The whole anti-Fc antibody is cleaved with pepsin to generate a bivalent F(ab)'₂ fragment. This fragment is typically affinity purified on a column comprising immunoglobulin proteins such as IgG that is immobilized on agarose. The fragment is then reduced to break the disulfide bond of the hinge region that connects the two Fab fragments resulting in a Fab' fragment with an exposed thiol group. This is typically accomplished by adding a mild reducing buffer to the affinity purified F(ab')₂ fragments such as a buffer comprising 0.01 M EDTA and 0.01M cysteine in phosphate buffer saline (PBS). The resulting thiol group readily reacts with a reactive group on a label to covalently attach the label to the fragment. Thus, a solution containing a label that has been chemically modified to contain a reactive group, using methods well known to one skilled in the art, is added to the solution of reduced Fab' fragments. This method is particularly useful for covalently attaching enzyme and other protein labels due to their size and the lack of exposed amine groups on the Fab fragments. One of skill in the art will appreciate that this method requires the use of Fab' fragments as apposed to Fab fragments due to the disulfide bonds of the Fab' fragment and that the use of the enzyme papain or the like results in such a fragment.

An alternative labeling of monovalent antibody fragments is also accomplished in a solution. The method comprises the steps:
a) contacting a Fab fragment with a solution comprising a label that contains a reactive group; and,
b) isolating labeled anti-Fc Fab fragment by size exclusion or affinity chromatography.

When a Fab fragment is to be labeled the whole antibody is cleaved with an enzyme, such as papain, to generate Fab monovalent fragments and the fragments are typically purified on an affinity column prior to addition of the label. The Fab fragments are optionally chemically modified to contain a reactive group. However, for covalently attaching reactive fluorophore labels it has been found that this modification of the fragment of non-antibody protein is not necessary. The reactive label, typically a fluorophore or hapten, are added to a solution of Fab fragments or non-antibody proteins and the labeling reagent is separated from excess label by size exclusion or affinity chromatorgraphy. The labeling reagents are then stored in an appropriate buffer.

Labeling in solution can have some drawbacks, especially when labeling Fab fragments with fluorophores. Thus, the Fab fragments used in a method of the present invention are preferably covalently attached to a fluorophore label when immobilized on an affinity column. The fragments are immobilized on an affinity column that comprises a protein that the fragment has affinity for, typically IgG, and after immobilization a reactive fluorophore is added to the column wherein the fragments are labeled and unreacted fluorophores pass through the column.

The use of this affinity chromatography method avoids the incorporation of label into the binding domain of the Fab fragment or non-antibody protein. When Fab fragments are labeled with fluorophores using this method unexpected advantages were obtained wherein the fluorescent signal form fragments labeled on a column are brighter than fragments labeled in solution when the fluorophore and ratio of fluorophore to labeling reagent are held constant. Without wishing to be bound by a theory it is possible that the decreased brightness observed from the fragments labeled in solution is due to quenching of fluorphores that are bound in or near the binding domain by the high concentration of amine groups in the binding domain. Thus, a preferred method for covalently attaching fluorphore labels to Fab fragments comprises the following steps:
a) cleaving an intact anti-Fc antibody with an enzyme that generates Fab fragments;
b) isolating the anti-Fc Fab fragments of step a);
c) contacting a matrix comprising intact immunoglobulin proteins or fragments thereof that specifically bind anti-Fc Fab fragments with a solution comprising said anti-Fc fragments of step b) wherein said Fab fragments are immobilized;
d) contacting said matrix of step c) with a solution comprising a fluorophore label that contains a reactive group;
e) washing said matrix to remove unbound label, and;
f) eluting said labeling reagent from said matrix whereby said labeling reagent is manufactured comprising a label and being isolated from other proteins and thereof.

The matrix is typically an agarose column that comprises either the Fc region, or the entire antibody provided that the antibody or fragment thereof is the same species and isotype that was used to produce the antibodies that the labeling reagent was generated from. However any matrix known to one skilled in the art can be used that allows for immobilization of labeling reagent and removal following attachment of the fluorophore label. Fab and Fab' fragments can both be labeled in this manner. However a free thiol group is not necessary and therefore Fab fragments are typically labeled using this method.

Enzyme or other protein labels may be covalently attached to Fab' fragments in solution utilizing the free thiol group of the Fab' fragment.

The attachment of the label to the fragments may result in multiple subsets that are distinguished by the.ratio of the label to the labeling reagent and the physical properties of the label. A labeling reagent subset as used herein refers to a discrete set of labeling reagents that are homogenous and can be distinguished from another subset of labeling reagent either by the physical properties of the label or the ratio of the label to labeling reagent. The physical properties include differences within a group of labels, such as emission spectra of fluorphores, or across groups of labels, such as the difference between an enzyme and a fluorophore. For fluorphare labels, the physical properties typically relates to the emission spectra, this includes modification of the same label, e.g. a cyanine with different substitutions that shifts the emission wavelength, or different fluorophores, e.g. a cyanine and a coumarin on the same labeling reagent. The difference in physical properties also includes the use of tandem dyes, which is specifically defined to include an energy transfer pair wherein one is a protein and the other is a fluorophore, or the pairing of other labels that are not necessarily energy transfer pairs. A few examples of labeling reagent subsets includes, but are not limited to, a first subset comprising a single fluorophore at a known ration attached to a anti-Fc Fab fragment; a second subset comprises the same fluorophore on the Fab fragment at a different known ration from the first subset, a third subset comprises the same fluorophore but that has a shifted wavelength due to a substitution on the fluorophore. Thus, the attachment of labels to the labeling reagents results in an extensive selection of subsets that when complexed with a target-binding antibody results in a unique method to detect one or multiple targets in a sample whereby the target is identified and quantitated.

### B. Immuno-labeled Complex

In one embodiment, subsets of labeling reagent are complexed with target-binding antibodies to produce subsets of immuno-labeled complex. As indicated above, such methods for forming the immuno-labeled complex comprise the following steps:
a) contacting a solution of target-binding antibodies with a labeling reagent subset, wherein said labeling reagent subsets are distinguished by i) ratio of label to labeling reagent or ii) physical properties of said label;
b) incubating said target-binding antibodies and said labeling reagent for a time period sufficient for one or more labeling reagents to form an immuno-labeled complex with a target-binding antibody wherein a region of said target binding antibody is selectively bound by labeling reagent;
c) optionally removing unbound labeling reagent by adding a capture reagent comprising immunoglobulin proteins or fragments thereof; and,
d) repeating said steps a), b), and c) to form individual or pooled subsets of immuno-labeling complexes wherein each subset is distinguished from another subset by i) a ratio of label to labeling reagent, or ii) a physical property of said label, or iii) a ratio of labeling reagent to said target-binding antibody, or iv) by said target-binding antibody, wherein said labeling reagent is an anti-Fc region Fab or Fab¹ fragment.

A particular advantage for the use of labeling reagent in accordance with the present invention to label target-binding antibodies is that the process is relatively insensitive to the solution the antibodies are in. Due to the physical nature of the labeling reagents, small monovalent fragments, the reagents do not cross-link and fall out of solution in the presence of high concentration of proteins. For this reason, target-binding antibodies can be complexed when present in ascites fluid, tissue culture supernatant, serum or other solutions where there is a high concentration of proteins. This eliminates the need to purify target-binding proteins prior to labeling.

When preparing the immuno-labeled complex using purified target-binding antibody, stock solutions of both the labeling reagent and the target-binding antibody are typically near 1 mg/mL in an appropriate buffer, although more or less concentrated solutions are also suitable. Generally, the labeling reagent is mixed in a molar ratio of at least one to 50 moles of labeling reagent to one mole of the target-binding antibody to be complexed. More commonly a ratio of at least one to as many as 10 moles of labeling reagent per mole of target-binding antibody is combined. With an anti-Fc region Fab to a target-binding antibody, a molar ratio of approximately 2 to 10 is typical, more typically 3 to 5 (particularly for complexes in which the labeling reagent has been labeled while immobilized on an affinity matrix). The ease of formation of the complex permits rapid optimization of the complex and assessment of the effect of variation in experimental parameters. A particularly unique advantage of the invention is that the stoichiometry of the complex is easily adjusted to provide complexes with different ratios of labeling reagent to target-binding antibody, and thus there is control over the ultimate detectability of the target in the sample. Complexes that have been labeled with the same dye but at different molar ratios can be separately detected by the differences in their intensities.

Complex formation appears to occur almost within the mixing time of the solutions (< 1 minute) but the reaction typically is allowed to proceed for at least 5 minutes and can be longer before combining the immuno-labeled complex with the sample. Although complex formation can be reversed by addition of an unlabeled antibody that contains the same binding region, reversibility is very slow; furthermore, following binding of the immuno-labeled complex to a target in a sample, the sample can be "fixed" using aldehyde-based fixatives by methods that are commonly practiced by those skilled in the art of immunolabeling.

The labeling process optionally further comprises the addition of a capture component to remove excess labeling reagent. For applications in which immunolabeling complexes of multiple primary antibodies from the same species (e.g. mouse monoclonal antibodies) or cross-reacting species (e.g. mouse and human antibodies) are to be used simultaneously or sequentially, it is necessary to quench or otherwise remove any excess labeling reagent by use of a capture component or by other means to avoid inappropriate labeling of the sample. The most effective capturing components to capture excess labeling reagent are those that contain the binding site of the labeling reagent but are themselves not labeled, preferably an antibody or antibody fragment. Capture components may be free in solution or immobilized on a matrix, such as agarose, cellulose, or a natural or synthetic polymer, to facilitate separation of the excess capture component from the immuno-labeled complex. The capture component is optionally attached to a microsphere or magnetic particle. However, separation of excess labeling reagent is not essential for successful utilization of the invention, particularly when using a single target-binding antibody.

As indicated above, the steps of the labeling process for the target-binding antibodies can be repeated to form discrete immuno-labeled complex subsets that can be used individually or pooled in an assay to detect individual or multiple targets. As used herein the term immuno-labeled complex subsets refers to subsets that are distinguished from each other i) a ratio of label to labeling reagent, or ii) a physical property of the label, or iii) a ratio of labeling reagent to the target-binding antibody, or iv) by the target-binding antibody, or a combination thereof. For example a panel of subsets may comprise a target-binding antibody that is bound by a labeling reagent comprising a subset of different ratios of the same label on the labeling reagent resulting in a discrete subset of immuno-labeled complexes. This subset of immuno-labeled complexes can be used individually wherein a target is identified by the intensity of the detectable label or used in combination with another subset of immunocomplexes that differ in the target-binding antibody to identify multiple targets.

Immuno-labeled complex(es) obtained in accordance with the claimed invention can be used in a wide range of immunoassays, essentially in any assay a traditional secondary antibody is used including some assays that secondary antibodies are not used because of their size and ability to cross-link. Examples of such assays used to detect a target in a sample include immunoblots, direct detection in a gel, flow cytometry, immunohistochemistry, confocal microscopy, fluorometry, ELISA and other modified immunoassays. Methods of use of immuno-labeled complexes do not form part of the claimed invention, but some further discussion of these is included below.

A sample is incubated with a preformed immuno-labeled complex prepared in accordance with the invention comprising a labeling reagent and a target-binding antibody. Alternatively, subsets of labeling reagents bound to the same target-binding antibody can be used to identify and provide additional information about such targets. For example, subsets of labeling reagent can be employed wherein two discrete subsets are provided each with a distinct fluorophore label that is distinguished by its emission spectra, e.g. one that emits in the green spectra and one that emits in the red spectra. The labeling reagent subsets are then added to a solution of target-binding antibody in a controlled ratio, e.g. two parts one labeling reagent (green emission) and one part the other labeling reagent (red emission) per target binding antibody. In this way the immuno-labeled complexes can be used to detect a target. If another immuno-labeled complex were added to the sample the original target could be distinguished from the subsequently detected target.

Multiple targets include the discrete epitope that the target-binding antibody has affinity for as well as molecules or structures that the epitiope is bound to. Thus, multiple target identification includes phenotyping of cells based on the concentration of the same cell surface marker on different cells. In this way multiple target identification is not limited to the discrete epitope that the target binding antibody binds, although this is clearly a way that multiple targets can be identified, i.e. based on the affinity of the target-binding antibody.

Therefore, a method for detecting multiple targets in a sample may comprise the following steps:
a) contacting said sample with a solution comprising a pooled subset of immuno-labeled complexes, prepared in accordance with the invention wherein each subset is distinguished from another subset by i) a ratio of label to labeling reagent, or ii) a physical property of said label, or iii) a ratio of labeling reagent to said target-binding antibody, or iv) by said target-binding antibody;
b) incubating said sample for a time sufficient to allow said immuno-labeled complexes to selectively bind to their target; and,
c) illuminating said immuno-labeled complex whereby said targets are detected.

A selected target-binding antibody and a subset of labeling reagent are incubated to form an immuno-labeled complex subset. This procedure is repeated to form a panel of immuno-labeled complex subsets that may be pooled and added to a sample. Alternatively each immuno-labeled complex subset is added stepwise to a sample. The immuno-labeled

complex subsets are distinguished by four characteristics resulting in an infinite number of immuno-labeled complex subsets. First (i) the subsets can be distinguished by the target-binding antibody that is determined by the end user for the information that is desired from a sample. This means that each subset is distinguished based on the affinity of the target-binding antibody. The target-binding antibody typically distinguishes immuno-labeled complexes when multiple targets are identified, however this is normally combined with another characteristic to gain information form a sample or increase the number of targets that can be detected at one time. The second (ii) distinguishing feature used is the ratio of label to labeling reagent, as discussed in detail above. A subset based on this feature would have for example a ratio of two fluorophore per each labeling reagent. The third (iii) distinguishing feature is the ratio of labeling reagent to target-binding antibody. This is accomplished using a controlled concentration of target-binding antibody mixed with a controlled concentration of a labeling reagent subset and the subset would comprise a target-binding antibody that is bound by a discrete number of labeling proteins. The fourth (iv) feature is the physical feature of the label. Typically this refers to the physical properties of the fluorophore labels wherein a subset of this group is distinguished by the label itself such as a green emitting fluorophore compared to a red emitting fluorophore. One of skill in the art will appreciate that while immuno-labeling complex subsets can be distinguished based on one feature the subsets are typically, and most useful, when discretely identified based on a combination of the distinguishing characteristics.

Another example of detection of multiple targets utilizes the following immuno-labeled subsets, all of which comprise a different target-binding antibody but differ in the label and ratio of label. The first subset comprises a fluorophore label that emits red-fluorescent light, a second subset comprises a fluorophore label that emits green fluorescent light, a third subset comprises a ratio of 1:1 red to green fluorophore label; a fourth subset comprises a ratio of 2:1 red to green fluorophore label and a fifth subset comprises a ratio of 1:2 red to green fluorophore label. These subsets allow for the simultaneous detection of five targets in a sample. This aspect of the present invention is particularly important due to the limited range of fluorophores available wherein the labeling reagents can be utilized to increase the number of targets that can be detected at one time. One of skill in the art can appreciate that these subsets could be expanded by altering the ratio of label to labeling reagent instead of just the ratio of labeling reagent to target-binding antibody. This same methodology can also be applied to a single fluorophore label wherein the ratios are altered and a target is detected based on the intensity of the signal instead of the color and the ratio of the color to another color.

Following the formation of the immuno-labeled complex subsets the subsets can be pooled and added to a sample or added stepwise to a sample, either of which is determined by the end user and the particular assay format. This provides for maximum flexibility and ease of determining multiple targets in a sample.

Determination of multiple targets in a sample may be carried out using the flow cytometry assay format. Traditionally targets identified using flow cytometry used either directly labeled primary antibody or labeled microspheres that were covalently attached to a primary antibody wherein the microsphere is the label. Examples include the fluorescent encapsulated microsphere beads sold by Luminex. Use of immuno-labeled complexes prepared in accordance with the present invention overcomes both the need for directly labeled primary antibody and the need for expensive microspheres.

Thus, a method for determining identity and quantity of targets in a sample by detecting multiple targets may comprise the following steps:
a) contacting a population of cells in a sample with a solution comprising a pooled subset of immuno-labeled complexes, prepared in accordance with the invention, wherein each subset is distinguished from another subset by i) a ratio of label to labeling reagent, or ii) a physical property of said label, or iii) a ratio of labeling reagent to said target-binding antibody, or iv) by said target-binding antibody;
b) incubating said cells for a time period sufficient to allow said immuno-labeled complexes to bind said targets;
c) passing said incubated population of cells through an examination zone; and
d) collecting data from said cells that were passed through said examination zone, wherein said multiple targets are detected whereby the identity and quantity of said targets is determined.

In one aspect, a target-binding antibody is pre-complexed to the target-binding antibody to form a subset and that subset or a panel of subsets are added to a sample, that are typically distinguished by the target binding antibody. This method then avoids the need for a directly labeled primary. Secondly, when the panel of subsets is distinguished, for example, by the ratio of label to labeling reagent or the ratio of labeling reagent to target-binding antibody the immuno-labeled complex can function similar to the microsphere beads of Luminex. For example, this is accomplished wherein three immuno-labeled complex subsets are distinguished by the target binding antibody and the fluorophore attached to the labeling reagent and within one of the subsets is another set of subsets that are distinguished based on the ratio of label to labeling reagent. In this way three different epitopes are detected and one of the epitopes is further distinguished and a phenotype distinction made based on the intensity of the signal generated from the labeled-immuno complex subsets based on the ratio of fluorophore to labeling reagent. This determination of targets is facilitated when a population of cells or cellular organelles is passed through the examination zone of a flow cytometer wherein the fluorescent signal and intensity is recorded for each cell resulting in a histogram of the cell population or cellular organelles based on the detected epitopes.

Additional detection reagents may be combined with the sample concurrently with or following the addition of immuno-labeled complex subsets. Such additional detection reagents include, but are not limited to reagents that selectively detect cells or subcellular components, ions, or indicate the cell viability, life cycle, or proliferation state. For example, the additional detection reagent is a labeled target-binding antibody that is directly or indirectly detectable and another additional detection reagent is a stain for nucleic acids, for F-actin, or for a cellular organelle.

### C. Applications

Immuno-labeled complexes prepared in accordance with the claimed invention have useful applications in basic research, high-throughput screening, immunohistochemistry, fluorescence in situ hybridization (FISH), microarray technology, flow cytometry, diagnostics, and medical therapeutics. They can be used in a variety of assay formats for diagnostic applications in the disciplines of microbiology, immunology, hematology and blood transfusion, tissue pathology, forensic pathology, and veterinary pathology. Such immuno-labeled complexes are particularly useful in the characterization and selection of optimized antibodies from hybridoma supernatants. Additionally, they can be used to deliver therapeutics to a specific target. In general, they provide a versatile and convenient method to enhance any assay that uses an antibody as part of its detection methodology.

Immuno-labeled complexes prepared in accordance with the claimed invention can be used to study biological phenomena, such as, for example, cell proliferation, signal transduction in cells, or apoptosis. For illustration purposes only and not limitation, one could study thymidine analog 5-bromo-2'-deoxyuridine (BrdU) incorporation. BrdU is a marker for both cell proliferation and apoptosis, as it is readily incorporated into newly synthesized DNA that has progressed through the S-phase of the cell cycle and also into DNA break sites by deoxynucleotidyl transferase (TdT). Anti-BrdU antibodies are used to detect cells marked by BrdU incorporation. By being able to directly label the anti-BrdU antibodies, the current invention provides a convenient method to allow for detection of the incorporated BrdU by conventional immunohistochemistry or fluorescence, depending on detection method required.

Additionally, the current invention has the advantage of allowing staining for multiple targets in one cocktail, thereby reducing the need for more samples or processing steps per experiment. This is particularly important when analyzing precious samples (e.g., pediatric samples, leukocytes isolated from biopsies, rare antigen-specific lymphocytes and mouse tissues that yield a small number of cells). Although it is currently possible to simultaneously measure up to 11 distinct fluorescent colors through a convoluted series of novel developments in flow cytometry hardware, software, and dye chemistry, the use of these advances has been severely limited by the lack of commercial availability of spectrally distinct directly labeled primary and secondary antibodies. Although labeled secondary antibodies directed at individual isotype-specific targeting antibodies (e.g., anti-IgG1 isotype antibodies) exist, it is not possible to use this type of labeled antibody to detect more than one of the same isotype of an antibody (e.g., an IgG1 isotype antibody) in a single sample due to cross-reactivity. The current invention overcomes these limitations by providing for a convenient and extremely versatile method of rapidly labeling either small or large quantities of any primary antibody including primary antibodies of the same isotype to be used in, for example, multicolor flow cytometry and on Western blots. This advance in multicolor systems has a number of advantages over current two- and three-color flow cytometric measurements. For example, no combination of one-color stains can accurately enumerate or be used to isolate CD3⁺ CD4⁺ CD8⁺ T cells (excluding, for example CD3⁺ CD4⁺ CD8⁺T cells and small CD4⁺ monocytes). The use of cell membrane markers to study leukocyte composition in blood and tissue serves as an example of an analytical monoclonal antibody application, particularly in combination with flow cytometry. It is also the example most relevant to studies of the immune system, because the cellular composition of blood and lymphoid tissue provides a 'window', allowing the analysis and monitoring of the immune system.

The methods of the invention can also be used in immunofluorescence histochemistry. This technique involves the use of antibodies labeled with fluorophores to detect substances within a specimen. The pathologist derives a great deal of information of diagnostic value by examining thin sections of tissue in the microscope. Tissue pathology is particularly relevant to, for example, the early diagnosis of cancer or premalignant states, and to the assessment of immunologically mediated disorders, including inflammation and transplant rejection. The problems associated with immunofluorescence histochemistry, however, stem from the limitations of the methods currently available for use in such application. For example, directly labeling an antibody can result in antibody inactivation and requires a relatively large of amount of antibody and time to do the conjugation. It is also expensive and impractical to prepare directly labeled antibodies having variable degrees of label substitution. Similarly, indirect labeling of an antibody has problems, such as lack of secondary antibody specificity, and reliance upon primary antibody differences, including antibody isotypes and available fluorophores, to do multicolor labeling. Secondary antibody labeling is not practical where the primary antibody is from the same species or of the same isotypes. Combinations of fluorophores or other detectable labels on the same target-binding antibody, which can be readily prepared in multiple mixtures by the methods on this invention, greatly increase the number of distinguishable signals in multicolor protocols. Lack of secondary antibody specificity arises when the specimen containing the targeted moiety and target-binding antibody are from homologous species. For example, BrdU-labeled DNA in rodent tissue is detected by immunohistochemical staining. The target-binding antibody is conventionally mouse anti-BrdU, and the detecting antibody system uses an anti-mouse immunoglobulin antibody, labeled with fluorescein. Because there is homology between mouse immunoglobulin and immunoglobulins from a number of rodent species (for example, rats, mice, hamsters, etc.), the detecting antibody not only binds to the target-binding antibody, but also nonspecifically binds to immunoglobulin in the tissue. The current invention eliminates this problem by pre-forming the immunolabeling complex and allows for a simple, rapid and convenient method to proceed with labeling with two, three or more fluorescent antibodies in one experiment. Very significantly, it can always be used with primary antibodies of either the same or different isotype, and always on tissue of the same or similar species as the primary antibody.

Immuno-labeled complexes prepared in accordance with the claimed invention also have application in the field of microarrays. Microarray technology is a powerful platform for biological exploration (Schena (Ed.), Microarray Biochip Technology, (2000)). Many current applications of arrays, also known as "biochips," can be used in functional genomics as scientists seek characteristic patterns of gene expression in different physiopathological states or tissues. A common method used in gene and protein microarray technology involves the use of biotin, digoxigenin (DIG), or dinitrophenyl (DNP) as an epitope or a "tag" such as an oligohistidine, glutathione transferase, hemagglutinin (HA), or c-myc. In this case a detectably labeled anti-biotin, anti-DIG, anti-DNP, anti-oligohistidine, anti-glutathione transferase, anti-HA, or anti-c-myc is used as the detection reagent. The instant Invention allows for the use of multiple fluorophore- or enzyme-labeled antibodies, thereby greatly expanding the detection modalities and also providing for enhanced multiplexing and two-dimensional analysis capabilities.

Similarly, the invention can be used with protein microarrays and on Western blots. Protein microarrays can provide a practical means to characterize patterns of variation in hundreds of thousands of different proteins in clinical or research applications. Antibody arrays have been successfully employed that used a set of 115 antibody/antigen pairs for detection and quantitation of multiple proteins in complex mixtures ( Haab et al., Genome Biology, 2, 4.1 (2001 )). However, protein microarrays use very low sample volumes, which historically have significantly limited the use of antibody technology for this application. The invention of the application readily overcomes this limitation and provides a means to label antibodies with the fluorescent dyes using a very low sample volume and to automate formation of the staining complex and the staining process.

Immuno-labeled complexes prepared in accordance with the claimed invention also provides a means for the specific detection, monitoring, and/or treatment of disease, in this regard the use of immunolabeling complexes to detect the presence of particular targets in vitro is contemplated. In such immunoassays, the sample may be utilized in liquid phase, in a gel, or bound to a solid-phase carrier, such as an array of fluorophore-labeled microspheres (e.g., U.S. Pat. No. 5,981,180 and 5,736,330). For example, a sample can be attached to a polymer, such as aminodextran, in order to link the sample to an insoluble support such as a polymer-coated bead, plate, or tube. For instance, but not as a limitation, in an in vitro assay, antibodies that specifically recognize an antigen of a particular disease can be used to determine the presence and amounts of this antigen.

Likewise, immunolabeling complexes prepared in accordance with the claimed invention can be used to detect the presence of a particular target in tissue sections prepared from a histological specimen. Preferably, the tissue to be assayed will be obtained by surgical procedures, e.g., biopsy. The excised tissue will be assayed by procedures generally known in the art, e.g. immunohistochemistry, for the presence of a desired target that is recognized by an immunolabeling complex, as described above. The tissue may be fixed or frozen to permit histological sectioning. The immunolabeling complex may be labeled, for example with a dye or fluorescent label, chemical, heavy metal or radioactive marker to permit the detection and localization of the target-binding antibody in the assayed tissue. In situ detection can be accomplished by applying a detectable immunolabeling complex to the tissue sections. In situ detection can be used to determine the presence of a particular target and to determine the distribution of the target in the examined tissue. General techniques of in situ detection are well known to those of ordinary skill. See, for example, Ponder, "Cell Marking Techniques and Their Application," in MAMMALIAN DEVELOPMENT: A PRACTICAL APPROACH, Monk (ed.), 115 (1987).

For diagnosing and classifying disease types, tissues are probed with an immuno-labeled complex, as defined above, that comprises a target-binding antibody to a target antigen associated with the disease, e.g., by immunohistochemical methods. Where the disease antigen is present in body fluids, such immuno-labeled complexes comprising a target-binding antibody to the disease antigen are preferably used in immunoassays to detect a secreted disease antigen target.

Detection can be by a variety of methods including, for example, but not limited to, flow cytometry and diagnostic imaging. When using flow cytometry for the detection method, the use of microspheres, beads, or other particles as solid supports for antigen-antibody reactions in order to detect antigens or antibodies in serum and other body fluids is particularly attractive. Flow cytometers have the capacity to detect particle size and light scattering differences and are highly sensitive fluorescence detectors. Microfluidic devices provide a means to perform flow-based analyses on very small samples.

Alternatively, one can use diagnostic imaging. The method of diagnostic imaging with radiolabeled antibodies is well known. See, for example, Srivastava (ed.), RADIOLABELED MONOCLONAL ANTIBODIES FOR IMAGING AND THERAPY, Plenum Press (1988 ); Chase, "Medical Applications of Radioisotopes," in REMINGTON'S PHARMACEUTICAL SCIENCES, 18th Edition, Gennaro et al. (eds.) Mack Publishing Co., 624 (1990); and Brown, "Clinical Use of Monoclonal Antibodies," in BIOTECHNOLOGY AND PHARMACY, Pezzuto et al. (eds.), Chapman & Hall, 227 (1993). This technique, also known as immunoscintigraphy, uses a gamma camera to detect the location of gamma-emitting radioisotopes conjugated to antibodies. Diagnostic imaging is used, in particular, to diagnose cardiovascular disease and infectious disease.

Thus, use of immuno-labeled complexes prepared in accordance with the claimed invention to diagnose cardiovascular disease is contemplated. For example, immuno-labeled complexes comprising anti-myosin antibodies can be used for imaging myocardial necrosis associated with acute myocardial infarction. Immuno-labeled complexes comprising antibodies that bind platelets and fibrin can be used for imaging deep-vein thrombosis. Moreover, Immuno-labeled complexes comprising antibodies that bind to activated platelets can be used for imaging atherosclerotic plaque.

Immuno-labeled complexes prepared in accordance with the claimed invention also can be used in the diagnosis of infectious diseases. For example, immuno-labeled complexes comprising antibodies that bind specific bacterial antigens can be used to localize abscesses. In addition, immuno-labeled complexes comprising antibodies that bind granulocytes and inflammatory leucocytes can be used to localize sites of bacterial infection. Similarly, the immuno-labeled complexes of the present invention can be used to detect signal transduction in cells, the products of signal transduction, and defects, inhibitors, and activators of signal transduction.

Numerous studies have evaluated the use of antibodies for scintigraphic detection of cancer. Investigations have covered the major types of solid tumors such as melanoma, colorectal carcinoma, ovarian carcinoma, breast carcinoma, sarcoma, and lung carcinoma. Thus, the present invention contemplates the detection of cancer using immuno-labeled complexes comprising antibodies that bind tumor markers (targets) to detect cancer. Examples of such tumor markers include carcinoembryonic antigen, [alpha]-fetoprotein, oncogene products, tumor-associated cell surface antigens, and necrosis-associated intracellular antigens. In addition to diagnosis, antibody imaging can be used to monitor therapeutic responses, detect recurrences of a disease, and guide subsequent clinical decisions and surgical procedures. In vivo diagnostic imaging using fluorescent complexes that absorb and emit light in the near infrared (such as those of the Alexa Fluor 700 and Alexa Fluor 750 dyes) is also known.

### EXAMPLES

The following examples illustrate the invention. The examples should not be construed as limiting the invention as the examples merely provide specific methodology useful in understanding and practising the invention.

### Example 1. Preparation of Fc antigen

Purified mouse and rabbit IgG was fragmented with the proteolytic enzyme papain (CURRENT PROTOCOLS IN CELT BIOLOGY, 16.4.1-16.4.10 (2000)). A 12 mL solution of mouse IgG was prepared at ~2 mg/mL in phosphate-buffered saline (PBS). A solution containing 0.1 mg of papain in digestion buffer (PBS, 0.02 M EDTA, 0.02 M cysteine) was added to the antibody and allowed to react at 37°C for 16 hours. The digestion was terminated by the addition 20 µL of 0.3 M iodoacetamide in PBS. The fragments were dialyzed against 2 L of PBS for 16 hours at 4°C. The Fc fragment was purified on a protein G-Sepharose CL-4B column. The bound fraction containing the Fc fragment was eluted from the column using 50-100 mM glycine/HlI buffer, pH 2.5-2.8. The eluate was collected in 1 mL fractions. The pH of the protein fractions was immediately raised to neutral by addition of 100 µL of either 500 mM phosphate or Tris buffer, pH 7.6, to each 1 mL fraction. The solution was then loaded onto a Sephacryl S-200 Superfine size-exclusion column and fractions corresponding to a molecular weight of -50 kDa were collected and analyzed by SDS-PAGE and HPLC.

### Example 2. Production of Anti-Fc antibodies.

Polyclonal antibodies specific for the Fc region of an antibody were raised in goats against the purified FC region of an antibody from a different species (Example 1). Methods of immunizing animals are well known in the art, and suitable immunization protocols and immunogen concentrations can be readily determined by those skilled in the art (Current Protocols in Immunology 2.4.1-9 (1995); ILAR Journal 37, 93 (1995)). Briefly, individual goats were immunized with purified mouse Fc or purified rabbit Fc fragments. The initial immunization in 50% Freund's complete adjuvant (1000 µg conjugate (half subcutaneous, half intramuscularly)) was followed by 500 [micro]g conjugate per goat in Freund's incomplete adjuvant two and four weeks later and at monthly intervals thereafter. Antibodies were purified from serum using protein A-Sepharose chromatography. Antibodies against mouse Fc isotypes can be prepared by starting with isotype-selected mouse Fc antigens. Rabbits have a single Fc isotype. Characterization of the selectivity and cross-reactivity of isotype-specific antibodies is by standard techniques, including HPLC.

### Example 3. Preparation of Fab fragments.

Fragmentation of the goat anti-(mouse Fc) antibody to the monovalent Fab fragment was carried out using the proteolytic enzyme, papain, as described in Example 1. Following dialysis against PBS, the Fab fragment was purified on a protein A-Sepharose CL-4B column. The unbound fraction containing the Fab fragment and the papain was collected. This solution was then loaded onto a Sephacryl S-200 Superfine size-exclusion column and fractions corresponding to a molecular weight of ~50 kDa were collected and analyzed by SDS-PAGE. The Fab fragments of goat anti-(rabbit Fc) can be prepared similarly.

### Example 4. Preparation of the labeled antibody immunoglobulin-binding protein in homogeneous solution.

Conjugates of antibody immunoglobulin-binding protein with low molecular weight dyes and haptens such as biotin or digoxigenin are typically prepared from succinimidyl esters of the dye or hapten, although reactive dyes and haptens having other protein-reactive functional groups are also suitable. The typical method for protein conjugation with succinimidyl esters is as follows. Variations in molar ratios of dye-to-protein, protein concentration, time, temperature, buffer composition and other variables that are well known in the art are possible that still yield useful conjugates.

A protein solution of the Fab fragment of goat anti-(rabbit Fc) is prepared at -10 mg/mL in 0.1 M sodium bicarbonate (pH -8.3). The labeling reagents are dissolved in a suitable solvent such as DMF at -10 mg/mL. Predetermined amounts of the labeling reagents are added to the protein solution with stirring. A molar ratio of 10 moles of dye to 1 mole of protein is typical; though the optimal amount can be varied with the particular labeling reagent, the protein being labeled and the protein's concentration. The optimal ratio was determined empirically. When optimizing the fluorescence yield and determining the effect of degree of substitution (DOS) on the conjugate's brightness, it is typical to vary the ratio of reactive dye to protein over a several-fold range. The reaction mixture is incubated at room temperature for a period that is typically one hour or on ice for several hours. The dye-protein conjugate is typically separated from unreacted reagents by size-exclusion chromatography, such as on BIO-RAD P-30 resin equilibrated with PBS. The initial, protein-containing band is collected and the DOS is determined from the absorbance at the absorbance maximum of each fluorophore, using the extinction coefficient of the free fluorophore. The DOS of nonchromophoric labels, such as biotin, is determined as described in Haugland ( Haugland et al., Meth. Mol. Biol. 45, 205 (1995); Haugland, Meth. Mol. Biol. 45, 223 (1995); Haugland, Meth. Mol. Biol. 45, 235 (1995); Haugland, Current Protocols in Cell Biol. 16.5.1-16.5.22 (2000 )). Using the above procedures, conjugates of goat anti-(mouse Fc) and goat anti-(rabbit Fc) were prepared with several different Alexa Fluor dyes, with Oregon Green dyes, with biotin-X succinimidyl ester, with desthiobiotin-X succinimidyl ester, with succinimidyl 3-(2-pyridyldithio)propionate (SPDP) and with succinimidyl trans-4-(maleimidylmethyl)cyclohexane-1-carboxylate (SMCC).

In addition, labeling proteins (goat Fab anti-(mouse Fc), goat Fab anti-(mouse lambda light chain), goat Fab anti-(mouse kappa light chain), conjugated to the detectable labels of R-phycoerythrin (R-PE), allophycocyanin (APC), tandem conjugates of phycobiliproteins with chemical dyes including several Alexa Fluor dyes, horseradish peroxidase (HRP), Coprinus cinereus peroxidase, Arthromyces ramosus peroxidase, glucose oxidase and alkaline phosphatase (AP) can be prepared by standard means (Haugland et al., Meth. Mol. Biol. 45, 205 (1995); Haugland, Meth. Mol. Biol. 45, 223 (1995); Haugland, Meth. Mol. Biol. 45, 235 (1995 ); Haugland, Current Protocols in Cell Biol 16.5.1-16.5.22 (2000 )).

### Example 5. Preparation of the labeled antibody immunoglobulin-binding protein while bound to an affinity matrix.

Unlabeled Fab fragment for goat anti-(mouse Fc) (prepared as in Example 3) was bound to agarose-immobilized mouse IgG for one hour. Following a wash step with bicarbonate buffer, pH 8.3, the complex of immobilized IgG and unlabeled Fab was labeled for one hour at room temperature with the succinimidyl ester of the amine-reactive label. Unconjugated dye was eluted with bicarbonate buffer, and then the covalently labeled Fab fragment was eluted with 50-100 mM glycine/HCl buffer, pH 2.5-2.8. The eluate was collected in 1 mL fractions. The pH of the protein fractions was immediately raised to neutral by addition of 100 µL of either 500 mM phosphate or Tris buffer, pH 7.6, to each 1 mL fraction. Variations of the reagent concentrations, labeling times, buffer composition, elution methods and other variables are possible that can yield equivalent results. Conjugates of the Fab fragment of goat anti-(rabbit Fc) may be prepared similarly.

### Example 6. Comparison of the Alexa Fluor 488 dye-labeled Fab fragments of goat anti-(mouse Fc) prepared as in Example 4 and as in Example 5.

Conjugates of the Fab fragment of goat anti-(mouse Fc) with the Alexa Fluor 488 succinimidyl ester were separately prepared, as described in Examples 4 and 5. The conjugates had estimated degrees of substitution of -1.9 (labeled as in Example 4) and -3.0 (labeled as in Example 5), respectively, and virtually identical absorption and emission spectral maxima. When excited at 488 nm, conjugates prepared using the fragment prepared as described in Example 5 were about 3.2-times more fluorescent than using the fragments that were prepared in Example 4 (Figure 8) as detected by flow cytometry when bound to CD3 on Jurkat T cells. Similar results were observed with other dyes.

### Example 7. Preparation of an immunolabeling complex on a very small scale.

Submicrogram quantities of a target-binding antibody were complexed with submicrograms of a labeling protein in varying molar ratios of between about 1:1 and 1:20 to prepare an immunolabeling complex that was suitable for staining a sample. For instance, 0.1 µg of mouse monoclonal anti-α-tubulin in 1 µL PBS with 0.1% BSA was complexed with 0.5 µg of the Alexa Fluor 488 dye-labeled Fab fragment of goat anti-(mouse Fc) (prepared as in Example 4) or with 0.1 µg of the Alexa Fluor 488 dye-labeled Fab fragment of goat anti-(mouse Fc) (prepared as in Example 5) in 5 [micro]L of PBS for 10 minutes at room temperature. The immunolabeling complex can be used immediately for staining tubulin in fixed-cell preparations or any excess unbound Alexa Fluor 488 dye-labeled Fab fragment of goat anti-(mouse Fc) in the immunolabeling complex can be captured with non-immune mouse IgG (Example 8) for combination with other antibody conjugates, including those of targeting antibodies that have been directly conjugated to other labels. In the case of a mouse (or rat) monoclonal antibody, it is preferred to use a labeled protein that is selective for the specific isotype of the primary antibody (e.g. anti-(mouse IgG₁) for a mouse

IgG₁ isotype primary antibody). Although some cross-reactivity for other mouse (or rat) isotypes was observed using a goat antibody that was selective for mouse IgG1 isotype monoclonal antibodies, routine and optimal use for labeling unmatched mouse isotypes required greater amounts of immunolabeling complexes and was somewhat less reliable.

### Example 8. Capturing excess immunoglobulin-binding protein by a capturing component.

Immunolabeling complexes were prepared as described in Example 7. To the immunolabeling complex was added to each tube 25 µL of a 14.1 mg/mL stock solution of unlabeled mouse IgG to capture excess immunolabeling complexes. As shown in Figure 1, not all of the immunoglobulin-binding protein was necessarily complexed with the target-binding antibody to form an immunolabeling complex. Consequently, particularly for applications in which labeling complexes of multiple primary antibodies from the same species (e.g. mouse monoclonal antibodies) or crossreacting species (e.g. mouse and human antibodies, Figure 2, Table 1) were to be used simultaneously or sequentially, it is necessary to quench or otherwise remove any excess immunoglobulin-binding protein by use of a capturing component or by other means to avoid inappropriate labeling of the sample. The most effective capturing component to capture excess immunoglobulin-binding protein is one that contains the binding site of the targeting agent. For instance, whole mouse IgG or mouse serum was shown to be an effective and inexpensive reagent when the immunoglobulin-binding protein was bound to a segment of a mouse monoclonal antibody. The mouse IgG was added in excess to the amount of immunoglobulin-binding protein and incubated for a period of approximately 1-5 minutes, or longer.

It is preferred to prepare the immunolabeling complex and then add the capturing component shortly before the experiment. The rapid quenching effect permits this to be done within minutes of performing labeling of the sample by the immunolabeling complex. If desired, the excess capturing component can be removed following labeling of the sample by a simple wash step. Alternatively, fixation of the stained sample by aldehyde-based fixatives or other reagents or methods subsequent to incubation with the immunolabeling complex can provide permanent immobilization of the immunolabeling complex on its target in the sample. As an alternative to adding a soluble capturing component to the immunolabeling complex, the capturing component can be immobilized on an insoluble matrix such as agarose and the immunolabeling complex contacted with that matrix. A preferred matrix when labeling mouse antibodies to mouse antigens is mouse IgG immobilized on agarose. Excess labeled anti-rabbit antibodies can be captured using rabbit IgG that is free in solution or immobilized. Alternatively, the immunolabeling complex can be separated from any capturing component by chromatographic or electrophoretic means.

### Example 9. HPLC analysis of a labeling complex.

In order to analyze the success and extent of complex formation of the labeling protein with the target-binding antibody, size exclusion HPLC of the samples was performed. For instance, a complex of Alexa Fluor 488 dye-labeled goat Fab anti-(mouse Fc) with a monoclonal mouse anti-tubulin in molar ratios of approximately 1:1, 3:1, 5:1 and 10:1. These were separated by analytical HPLC using a BioSep S-3000 column and eluting with 0.1 M NaPi, 0.1 M NaCl, pH 6.8, at a flow rate of 0.25 mLs/min. An example of the separation using the 5:1 molar ratio (Figure 6) demonstrates that, using this molar ratio, formation of the labeled complex is essentially quantitative.

### Example 10. Cross-reactivity of goat Fab anti-(mouse Fc) to other species of IgG.

Microplates were equilibrated overnight with IgG from a mouse or non-mouse species, and then further blocked with BSA. Variable amounts of the biotinylated Fab fragment of goat anti-(mouse Fc) were added to each well and allowed to bind. After washing, streptavidin-HRP and the Amplex Red peroxidase substrate were added. HRP activity was detected by the addition of H₂O₂ using the Amplex Red Peroxidase Assay Kit (Molecular Probes, Inc., Eugene, OR). Reactions containing 200 µM Amplex Red reagent, 1 U/mL HRP and 1 mM H₂O₂ (3% solution) in 50 mM sodium phosphate buffer, pH 7.4, were incubated for 30 minutes at room temperature. Fluorescence was measured with a fluorescence microplate reader using excitation at 560 ± 10 nm and fluorescence detection at 590 ± 10 nm. Background fluorescence, determined for a no-H₂O₂ control reaction, was subtracted from each value (Table 1 and Figure 2). Table 1 shows that the goat anti-(mouse Fc) antibody because of the highly conserved structure of the Fc region of an antibody it can be used to complex other non-mouse antibodies, including rat, and human antibodies. The goat anti-mouse IgG antibody reaction with mouse antibody was set at 100% and the crossreacting antibodies were expressed as a percentage compared the mouse on mouse data. The data in Table 1 show that the Fab fragment of the goat anti-(mouse Fc) antibody of the current invention does not strongly bind to the goat or sheep Fc domain; however, one skilled in the art could generate antibodies that will react with the goat and sheep Fc domain or the Fc domain of any other species. Biotinylated Fab goat anti-(mouse Fc) was used in this example because it provided a convenient method to quantitate the amount of crossreactivity in a conventional method but it could have been accomplished using a fluorophore Fab labeled goat anti-(mouse Fc). It was demonstrated by HPLC (as in Example 10) that Alexa Fluor 488 dye-labeled goat anti-(rabbit Fc) bound to rabbit primary antibodies.

**Table 1. Cross-reactivity of goat anti-mouse IgG antibody with other non-mouse antibodies.**

| Species | Crossreactivity | % Fluorescence |
|---|---|---|
| Mouse | ++++ | 100 |
| Rat | +++ | 80.7 |
| Human | ++ | 66.7 |
| Rabbit | + | 16.9 |
| Goat | - | 6.5 |
| Sheep | - | 5.7 |

### Example 11. Determination of the optimal molar ratio of immunoglobulin-binding protein to target antibody using a microplate assay.

To 1.6 µg of mouse monoclonal anti-biotin (MW 145,000) in 8.0 µL PBS was added varying amounts of the Alexa Fluor 488 dye-labeled Fab fragment of goat anti-(mouse Fc) (MW 50,000) (prepared as in Example 4) to form an immunolabeling complex. After equilibration for 20 min, a 100 µL aliquot was added to a 96-well microplate coated with biotinylated BSA. After 30 minutes, the plates were washed and the residual fluorescence was quantitated using a fluorescence microplate reader using excitation at 485 +/- 10 nm and detecting emission at 530 +/- 12.5 nm. As shown in Figure 3, a molar ratio of the Alexa Fluor 488 dye-labeled Fab fragment of goat anti-(mouse Fc) to the anti-biotin between 5 to 20 was sufficient to form appreciably detectable complexes (Figure 3; fluorescence quantitated, performed in triplicate (circles); control experiments performed but without adding the primary anti-biotin antibody (solid squares)). A molar ratio of about 5 to about 10 was preferred for this pair of immunoglobulin-binding protein and target antibody. This ratio can be varied somewhat to increase or decrease the signal or to affect the consumption of valuable reagents. The weight ratio of immunoglobulin-binding protein to target-binding antibody is particularly affected by the actual molecular weight of the immunoglobulin-binding protein.

For instance, equal weights of the dye-labeled goat Fab anti-(mouse Fc) (prepared as in Example 5) and an intact mouse primary antibody, which corresponds to an approximately 3 to 1 molar ratio, usually yields suitable labeling complexes. Fluorescence intensity (or enzymatic activity) of the immunolabeling complex is readily adjusted by a corresponding adjustment of the amount of labeled Fab fragment used.

### Example 12. Dissociation rate of the immunolabeling complex.

A pre-equilibrated immunolabeling complex was prepared from 50 µg of an Alexa Fluor 488 dye-labeled Fab fragment of goat anti-(mouse Fc) and 15 µg of an anti-biotin monoclonal antibody (mAb). The immunolabeling complex was rapidly diluted with capturing component sufficient to give a 6.2 molar excess over the anti-biotin mAb. At various times, an aliquot was taken and added to a microplate well containing an excess of biotinylated BSA. After 30 minutes, the plates were washed and the remaining fluorescence was quantitated. Displacement of the labeling protein from the target-binding antibody through exchange was measured by any time-dependent decrease in fluorescence in the microplate well. For example the fragments prepared as described in Example 4 had 68 percent fragments bound to the target-binding antibody after 30 minutes compared to 87 percent of bound fragments that were prepared according to Example 5. One hour showed a similar decrease, 56 percent and 68 percent respectively. The labeling protein was shown to undergo a stable interaction with the target-binding antibody, with a lifetime for half exchange under these conditions of 3.5 hours. Dissociation rates were measured for labeling protein prepared according to Example 4 and for labeling protein prepared according to Example 5, demonstrating the greater stability of immunolabeling complexes made using the labeling proteins prepared according to Example 5.

The assay protocols described in the following examples do not form part of the claimed invention, but are included to illustrate use of immuno-labeled complexes prepared in accordance with the claimed invention.

### Example 13. Protocol for staining cultured cells with a single immunolabeling complex.

Culturable cells, such as bovine pulmonary artery endothelial cells (BPAEC), were grown on a 22 x 22 mm glass coverslip. The cells were fixed for 10 minutes using 3.7% formaldehyde in DMEM with fetal calf serum (FCS) at 37°C. The fixed cells were washed 3 times with PBS. The cells were permeabilized for 10 min with 0.02% Triton X-100 in PBS, washed 3X with PBS and blocked for 30 min with 1% BSA in PBS. Variations of the cell type and cell preparation, fixation, and permeabilization methods, including methods for antigen retrieval, are well known to scientists familiar with the art. An immunolabeling complex was prepared as described in Example 7. The immunolabeling complex was added directly to the fixed and permeabilized cells in an amount sufficient to give a detectable signal if there is a binding site for the primary antibody present in the sample. After an incubation period that was typically 10-60 minutes (usually about 15-30 minutes), the cells were washed with fresh medium and the labeling was evaluated by methods suitable for detection of the label. Staining by the immunolabeling complex can be additionally preceded, followed by or combined with staining by additional reagents, such as DAPI, which yields blue-fluorescent nuclei.

### Example 14. Protocol for staining cultured cells with multiple immunolabeling complexes.

Cells were fixed and permeabilized as described in Example 13. Multiple immunolabeling complexes were individually prepared from a variety of labeling proteins, according to the procedure described in Example 7. The multiple immunolabeling complexes were either used individually or sequentially to stain the cells, according to the procedure described in Example 13, or two or more immunolabeling complexes were formed then co-mixed in a single staining solution and used to simultaneously stain the sample. The optimal method for cell fixation and permeabilization and the best ratio for combination of the immunolabeling complexes are typically determined by preliminary experimentation using single immunolabeling complexes or multiple immunolabeling complexes used in combination. A first immunolabeling complex was prepared from an Alexa Fluor 488 dye-labeled Fab fragment of goat anti-(mouse Fc) and mouse monoclonal anti-α-tubulin, a second immunolabeling complex was prepared from an Alexa Fluor 568 dye-labeled Fab fragment of goat anti-(mouse Fc) and mouse monoclonal anti-vimentin (anti-vimentin was an ascites fluid preparation) and a third Immunolabeling complex was prepared from an Alexa Fluor 647 dye-labeled Fab fragment of goat anti-(mouse Fc) and mouse monoclonal anti-odc6 peptide antibody (Molecular Probes). Aliquots of the three different immunolabeling complexes were combined and used to stain BPAE cells for 30 minutes, washed with fresh medium and observed by fluorescence microscopy using optical filters appropriate for the three dyes. In this example, some cells showed cytoplasmic staining by the anti-vimentin antibody, nuclear staining by the anti-cdc6 peptide antibody and staining of mitotic spindles by the anti-α-tubulin antibody, indicative of a cell in mitosis. Staining by the immunolabeling complexes was additionally preceded, followed by or combined with staining by additional reagents, such as Alexa Fluor 350 phalloidin, which yielded blue-fluorescent actin filaments in the above example.

The immunolabeling complexes that are used in combination do not have to be targeted toward antibodies from the same species. For instance, complexes of Alexa Fluor 488 dye-labeled goat anti-(mouse IgG₁ Fc) with a mouse IgG₁ monoclonal target-binding antibody and an Alexa Fluor 594 dye-labeled goat anti-(rabbit Fc) with a rabbit primary target-binding antibody can be prepared and used in combined staining protocols.

### Example 15. Protocol for staining tissue with a single immunolabeling complex.

A mouse intestine cryosection (University of Oregon histology core facility), a cross-section of about 16 µm thickness, was mounted on a slide. The intestine was perfused and fixed with 4% formaldehyde prior to dissection, embedding, and sectioning. The tissue section was rehydrated for 20 minutes in PBS. An immunolabeling complex was prepared as described in Example 8. Briefly, 0.1 µg of mouse monoclonal anti-cdc6 peptide (a nuclear antigen) in 1 µL PBS with 0.1% BSA was complexed with 0.5 µg of the Alexa Fluor 350 dye-labeled Fab fragment of goat anti-(mouse IgG1 Fc) (prepared as in Example 4) in 5 µL of PBS for 10 minutes at room temperature. Excess Fab fragment of goat anti-(mouse IgG1 Fc) was captured with 25 µL of a 14.1 mg/mL stock of unlabeled mouse IgG. The tissue was permeabilized with 0.1% Triton X-100 for 10 min. The tissue was washed two times with PBS and was blocked in 1% BSA for 30 min. The immunolabeling complex was added directly to the tissue for 30 minutes and washed three times in PBS. The sample was mounted in Molecular Probes' Prolong antifade mounting medium and observed by fluorescence microscopy using optical filters appropriate for the Alexa Fluor 350 dye. Results showed that the mouse monoclonal anti-cdc6 peptide immunolabeling complex showed specific nuclear labeling in the mouse intestine tissue section. Variations of the tissue type and tissue preparation, fixation and permeabilization methods, mounting methods, including methods for antigen retrieval, are well known to scientists familiar with the art.

### Example 16. Staining of a tissue target in combination with tyramide signal amplification (TSA).

[0153] Mouse brain cryosections were labeled with a pre-formed complex of horseradish peroxidase (HRP)-labeled goat anti-(mouse IgG1 Fc) antibody and a mouse IgG1 monoclonal anti-(glial fibrillary acidic protein (GFAP)) prepared essentially as in Example 8 using a molar ratio of labeling protein to monoclonal antibody of 3. Staining of the mouse tissues was essentially as in Example 15. The staining localization and intensity was compared to that of (a) goat anti-mouse IgG HRP conjugate and mouse anti-GFAP, (b) the Alexa Fluor 488 dye-labeled Fab fragment of goat anti-(mouse IgG1 Fc) antibody complex of mouse anti-GFAP, (c) Alexa Fluor 488 goat anti-mouse IgG secondary antibody and mouse anti-GFAP, and (d) a direct conjugate of the Alexa Fluor 488 dye with mouse anti-GFAP. The HRP-conjugated probes were incubated with Alexa Fluor 488 tyramide using TSA Kit #2 (Molecular Probes, Inc.) according to standard procedures. The tissue staining patterns in each case were similar and consistent with the expected staining pattern of mouse anti-GFAP and staining was essentially free of nonspecific background. The relative fluorescence intensities of staining measured by digital imaging were sequentially: 541 relative intensity units for the HRP-goat anti-(mouse IgG₁ Fc) complex of mouse anti-GFAP and (using the combinations indicated by the letters above): (a) 539, (b) 234, (c) 294, and (d) 255 relative intensity units.

### Example 17. Staining of live cells by multiple immunolabeling complexes.

A first immunolabeling complex was prepared from an Alexa Fluor 488 dye-labeled Fab fragment of goat anti-(mouse IgG₁ Fc) and mouse monoclonal anti-(human CD8), a second immunolabeling complex was prepared from an R-phyaoerythrin-conjugated Fab fragment of goat anti-(mouse IgG₁ Fc) and mouse anti-(human CD3), and a third immunolabeling complex was prepared from an Alexa Fluor 647 dye-labeled Fab fragment of goat anti-(mouse IgG₁ Fc) and mouse anti-(human CD4). The complexes were prepared as described in Example 8 and were each blocked with 20 µg 1.3µL of 14.1 µg/mL) of mouse IgG for 10 minutes at room temperature. The first immunolabeling complex was added to 100 [micro]L of whole blood and incubated for 15 min. The cells were washed with PBS and 280.5 µL of the second immunolabeling complex was added and incubated for 15 min. The cells were again washed, and 46.2 µL of the third labeling complex was added and incubated for 15 min. After the final incubation, the red blood cells were lysed with cell-lysis buffer. The cells were resuspended in 1% formaldehyde/PBS and analyzed on a FACS Vantage flow cytometer using a 488 nm argon-ion laser for excitation of the first and second immunolabeling complexes and a 633 nm red He-Ne laser for excitation of the third immunolabeling complex (Figures 5a, 5b). The emission band pass filters used for selective detection of the dyes are 525 +/- 10 nm for the Alexa Fluor 488 (CD8), 585 +/-21 nm for R-PE (CD3) and 675 +/- 10 nm for the Alexa Fluor 647 dye (CD4). Figures 5a and 5b show that the instant invention can be used in a 3-color immunophenotyping experiment using peripheral blood lymphocytes. CD3-positive T cells were stained with the R-phycoerythrin-conjugated Fab fragment of goat anti-(mouse Fc) and mouse anti-(human CD3), upper left (UL) quadrant, Figure 5a. CD4-positive cells, a T cell subset, are identified using Alexa Fluor 647 dye-labeled Fab fragment of goat anti-(mouse IgG₁ Fc) and mouse anti-(human

CD4), UL quadrant, Figure 5b and CD8-positive T cells, a T cell subset, were identified using Alexa Fluor 488 dye-labeled Fab fragment of goat anti-(mouse IgG₁ Fc) and mouse monoclonal anti-(human CD8), lower right (LR) quadrant, Figure 5b.

Exposed antigens of live cells, including cultured cells and cells from biological fluids such as blood and cerebrospinal fluid can be simultaneously or sequentially stained by combinations of immunolabeling complexes, including antibodies to the same target labeled with two or more separately detectable immunoglobulin-binding proteins.

### Example 18: The dye-labeled Fab fragment of goat anti-(mouse Fc) can be utilized for the combinatorial labeling of primary antibodies, to generate a multitude of colored targets.

A first immuno-labeled complex was made by combining 2.5 µg Alexa Fluor 488 dye-labeled Fab fragment of goat anti-(mouse IgG1 Fc) with 0.5 µg mouse anti-human CD3 (Caltag at 200 µg/mL), according to the procedure described in Example 4. A second immunolabeling complex was made by combining 5.0 µg Alexa Fluor 647 dye-labeled Fab fragment of goat anti-(mouse IgG₁ Fc) with 0.5 µg mouse anti-human CD3, according to the procedure in Example 4. Each complex was separately incubated at room temperature for 5 minutes, and each complex was then separately combined with an excess of mouse IgG (14.1 mg/mL) for 5 min at room temperature to capture excess unbound dye-labeled Fab fragments. The two immunolabeling complexes were then added in different percentage combinations (see Table 2) to 100 µL of washed heparinized blood. The cells were incubated with the respective combinations of complexes for 20 min on ice. The red blood cells were then lysed with a cell-lysis buffer. The cells were resuspended in 1 % formaldehyde/PBS and analyzed on a FacVantage flow cytometer using a 48B nm argon 633 HeNe laser for excitation and a 530 +/-10 nm band pass emission filter (FL1), and a 640 long pass filter (FL4). Five samples of different combined percentages (Table 2) were compared by flow cytometry, with signals being collected in FL1 and FL4. To determine the percentage of cells detected with each type of emission, the FL1 and FL4 intensities for each percentage combination were normalized by dividing the FL1 and FL4 channel intensities for such combinations by the intensities of the 100% Alexa Fluor 488 dye- and 100% Alexa Fluor 647 dye-labeled cells, respectively.

**Table 2. Theoretical versus recovered dye-labeled Fab fragment of goal anti-(mouse IgG₁Fc) combinatorial experiment.**

| Experimentally mixed percentage of cells labeled with Alexa Fluor 488 dye-labeled Fab fragment of goat anti-(mouse IgG₁ Fc) | Recovered percentage of cells labeled with Alexa Fluor 488 dye-labeled Fab fragment of goat anti-(mouse IgG₁ Fc) | Experimentally mixed percentage of cells labeled with Alexa Fluor 647 dye-labeled Fab fragment of goat anti-(mouse IgG₁ Fc) | Recovered percentage of measured cells labeled with Alexa Fluor 647 dye-labeled Fab fragment of goat anti-(mouse IgG₁ Fc) |
|---|---|---|---|
| 100% | 100% | 0% | 0% |
| 75% | 81% | 25% | 14% |
| 50% | 63% | 50% | 38% |
| 25% | 35% | 75% | 73% |
| 0% | 0% | 100% | 100% |

### Example 19: The immunolabeling complex can be used to detect antigens on a Western blot

Bovine heart mitochondria were isolated (Hanson et al., Electrophoresis 22, 950 (2001)). The isolated mitochondria were resuspended to -10 mg/mL in 100 mM Tris-HCl, pH 7.8, 1 mM phenylmethylsulfonyl fluoride (a protease inhibitor), 2% SDS and insoluble material was removed by centrifugation for 10 minutes at 10,000 x g in a tabletop centrifuge. The protein concentration of the lysate was checked by the BCA assay (Pierce, Rockford, IL). Samples for gel electrophoresis were prepared by mixing lysate, water, and loading buffer to the appropriate concentrations (final concentration of loading buffer in samples: 58 mM Tris/HCl, 10% glycerol, 2% SDS, 0.02 mg/mL bromphenol blue, 50 mM DTT, pH 8.6). The samples were then heated to 90°C for 5 minutes before loading on the gel and separated on a 13% SDS-PAGE geL Two-fold serial dilution of the extracts ranging from 8 [micro]g of extract down to 0.03 µg were loaded on the SDS-PAGE gel. The proteins were transferred to PVDF membrane for 1.5 hours using a semi-dry transfer system according to manufacturer's directions (The W.E.P. Company, Concord, CA). The PVDF membrane was blocked for 1 hour in 5% milk.

Immunolabeling complexes were made with mouse monoclonal antibodies that recognize two different mitochondrial proteins. Alexa Fluor 647 dye-labeled Fab fragment of goat anti-(mouse IgG₁ Fc) (5 µL of a 1 mg/mL stock, prepared as in Example 4) was incubated with 21 µL (0.88 mg/mL) mouse anti-(CV-alpha) and Alexa Fluor 488 dye-labeled Fab fragment of goat anti-(mouse IgG1 Fc) (5 [micro]L of a 1 mg/mL stock, prepared as in Example 4) was incubated with 19 µL (0.88 mg/mL) mouse anti-(GIII-care2) (Molecular Probes, Eugene, OR). Following a 30 minute incubation, 25 µL of a 14.1 mg/mL stock of unlabeled mouse IgG was added to each tube. The immunolabeling complexes were then mixed together and brought up to 5 mL in 5% milk. The blot was incubated with the mixture of immunolabeling complexes for 1 hour at room temperature. The blot was washed twice for 5 seconds each with PBST (PBS with 0.1 % Tween) and once with PBST for 15 minutes. The blot was air dried and imaged on an EG&G Wallac Imager with the appropriate filters. The Western blot revealed two distinct bands of the appropriate molecular weight. The Western blot also showed that no cross-labeling of the antibodies occurred and the detection limit was 125 ng.

### Example 20: High-throughput screening of hybridomas for identifying high affinity and high IgG producers

Microplate wells are prepared containing both a fluorescent labeled antigen of one fluorescent color label and fluorescently labeled Fab fragments of goat anti-(mouse Fc) of a different fluorescent color made by the method described in Example 4 and 5. Hybridoma supernatant is harvested and added to the wells. If the hybridoma are producing the desired antibody, i.e. antibodies that bind to the labeled antigen, polarization of the fluorescence corresponding to the labeled antigen will allow visualization of those wells containing antigen specific antibody. In addition, the amount of IgG that the hybridomas produce, can be simultaneously identified by polarization of the fluorescence corresponding to the labeled Fab fragments. This method thus allows for both quantitation of the amount of antibody present in a specific amount of hybridoma supernatant and the affinity of the monoclonal antibodies for the antigen.

The reagents employed in the preceding examples are commercially available or can be prepared using commercially available instrumentation, methods, or reagents known in the art or whose preparation is described in the examples. It is evident from the above description and results that the subject invention is greatly superior to the presently available methods for determining the presence of a target in a biological sample. The subject invention overcomes the shortcomings of the currently used methods by allowing small quantities of antibodies to be labeled and in unlimited media while maintaining specificity and sensitivity.

## Claims

1. A method of labeling a target-binding antibody, wherein said method comprises the steps of:
a) contacting a solution of target-binding antibodies with a labeling reagent subset, wherein said labeling reagent subsets are distinguished by i) ratio of label to labeling reagent or ii) physical properties of said label;
b) incubating said target-binding antibodies and said labeling reagent subset for a time period sufficient for one or more labeling reagents to form an immuno-labeled complex with a target-binding antibody wherein a region of said target binding antibody is selectively bound by labeling reagent;
c) optionally removing unbound labeling reagent by adding a capture reagent comprising immunoglobulin proteins or fragments thereof; and,
d) optionally repeating said steps a), b), and c) to form a panel of immuno-labeled complex subsets wherein each subset is distinguished from another subset by i) a ratio of label to labeling reagent, or ii) a physical property of said label, or iii) a ratio of labeling reagent to said target-binding antibody, or iv) by said target-binding antibody,
wherein the method is **characterised in that** said labeling reagent is an anti-Fc region Fab or Fab' fragment.

2. The method according to Claim 1, wherein said target binding antibody is a murine monoclonal antibody, a rabbit polyclonal antibody or a goat polyclonal antibody.

3. The method according to Claim 2, wherein said target-binding antibodies are in a solution comprising serum proteins or ascites proteins.

4. The method according to any of Claims 1 to 3, wherein said label is selected from the group consisting of a chromophore, a fluorophore, a fluorescent protein, a phosphorescent dye, a tandem dye, a particle, a hapten, an enzyme and a radioisotope.

5. The method according to Claim 4, wherein said fluorophore is selected from the group consisting of a coumarin, a xanthene, a cyanine, a pyrene, a borapolyazaindacene, an oxazine, and derivatives thereof.

6. The method according to Claim 4, wherein, said fluorescent protein is a phycobiliprotein.

7. The method according to Claim 4, wherein said tandem dye is selected from the group consisting of a cyanine-phycobiliprotein derivative and xanthenephycobiliprotein derivative.

8. The method according to Claim 4, wherein said enzyme is selected from the group consisting of a peroxidase, a phosphatase, a glycosidase, and a luciferase.

9. The method of claim 4, wherein said particle is a microsphere or a quantum dot.

10. The method according to any preceding claim, wherein said method further comprises repeating said steps a), b), and c) to form subsets of immuno-labeled complexes wherein each subset is distinguished from another subset by i) ratio of label to labeling reagent or ii) physical properties of said label, or iii) a ratio of labeling reagent to said target-binding antibody or iv) by said target-binding antibody.

## Patentansprüche

1. Verfahren zur Markierung eines Target-Bindungs-Antikörpers, wobei das genannte Verfahren die folgenden Schritte umfasst:
a) das Kontaktieren einer Lösung von Target-Bindungs-Antikörpern mit einer Markierungsreagenz-Teilmenge, wobei die genannten Markierungsreagenz-Teilmengen unterschieden werden nach i) Verhältnis der Markierung zu der Markierungsreagenz oder ii) den physikalischen Eigenschaften der genannten Markierung;
b) das Inkubieren der genannten Target-Bindungs-Antikörper und der genannten Markierungsreagenz-Teilmenge über einen Zeitraum, der ausreicht, damit eine oder mehrere Markierungsreagenzien einen immunomarkierten Komplex mit einem Target-Bindungs-Antikörper bilden können, wobei eine Region des genannten Target-Bindungs-Antikörpers selektiv durch die Markierungsreagenz gebunden ist;
c) das optionale Entfernen ungebundener Markierungsreagenz durch Hinzufügen einer Erfassungsreagenz, die Immunoglobulinproteine oder Fragmente dieser umfasst; und
d) das optionale Wiederholen der Schritte a), b) und c), so dass ein Feld von immunomarkierten Komplexteilmengen gebildet wird, wobei sich die einzelnen Teilmengen voneinander unterscheiden durch i) ein Verhältnis der Markierung zu der Markierungsreagenz oder ii) eine physikalische Eigenschaft der genannten Markierung oder iii) ein Verhältnis der Markierungsreagenz zu dem genanntenTarget-Bindungs-Antikörper oder iv) durch den genannten Target-Bindungs-Antikörper, wobei das Verfahren **dadurch gekennzeichnet ist, dass** es sich bei der Markierungsreagenz um ein Antif-Fc Region Fab- oder Fab'-Fragment handelt.

2. Verfahren nach Anspruch 1, wobei es sich bei dem genanntenTarget-Bindungs-Antikörper um einen monoklonalen Maus-Antikörper, einen polyklonalen Kaninchen-Antikörper oder einen polyklonalen Ziegen-Antikörper handelt.

3. Verfahren nach Anspruch 2, wobei sich die genannten Target-Bindungs-Antikörper in einer Lösung befinden, welche Serumproteine oder Aszitesproteine umfasst.

4. Verfahren nach einem der Ansprüche 1 bis 3, wobei die genannte Markierung aus der Gruppe ausgewählt wird, die folgendes umfasst: ein Chromophor, ein Fluorophor, ein fluoreszierendes Protein, einen phosphoreszierenden Farbstoff, einen Tandemfarbstoff, ein Partikel, ein Hapten, ein Enzym und ein Radioisotop.

5. Verfahren nach Anspruch 4, wobei das genannte Fluorophor aus der Gruppe ausgewählt wird, die folgendes umfasst: ein Cumarin, ein Xanthen, ein Cyanin, ein Pyren, ein Borapolyazaindacen, ein Oxazin und Derivate dieser.

6. Verfahren nach Anspruch 4, wobei es sich bei dem genanntenfluoreszierenden Protein um ein Phycobiliprotein handelt.

7. Verfahren nach Anspruch 4, wobei der genannte Tandemfarbstoff aus der Gruppe ausgewählt wird, die ein Cyanin-Phycobiliprotein-Derivat und ein Xanthenphycobiliprotein-Derivat umfasst.

8. Verfahren nach Anspruch 4, wobei das genannte Enzym aus der Gruppe ausgewählt wird, die eine Peroxidase, eine Phosphatase, eine Glycosidase und eine Luciferase umfasst.

9. Verfahren nach Anspruch 4, wobei es sich bei dem genanntenPartikel um eine Mikrosphäre oder einen Quantenpunkt handelt.

10. Verfahren nach einem der vorstehenden Ansprüche, wobei das genannte Verfahren ferner eine Wiederholung der genannten Schritte a), b) und c) umfasst, so dass Teilmengen immunomarkierter Komplexe gebildet werden, wobei jede Teilmenge von einer anderen Teilmenge unterschieden wird durch i) das Verhältnis der Markierung zu der Markierungsreagenz oder ii) die physikalischen Eigenschaften der genannten Markierung oder iii) ein Verhältnis der Markierungsreagenz zu dem genanntenTarget-Bindungs-Antikörper oder iv) den genannten Target-Bindungs-Antikörper.

## Revendications

1. Procédé de marquage d'un anticorps de liaison à une cible, dans lequel ledit procédé comprend les étapes consistant à :
a) mettre en contact une solution d'anticorps de liaison à une cible avec un sous-ensemble de réactifs de marquage, dans lequel lesdits sous-ensembles de réactifs de marquage se distinguent par i) le rapport entre traceur et réactif de marquage ou ii) des propriétés physiques dudit traceur ;
b) faire incuber lesdits anticorps de liaison à une cible et ledit sous-ensemble de réactifs de marquage pendant une période de temps suffisante pour qu'un ou plusieurs réactifs de marquage forment un complexe immuno-marqué avec un anticorps de liaison à une cible, dans lequel une région dudit anticorps de liaison à une cible est sélectivement fixée par un réactif de marquage ;
c) éliminer en option le réactif de marquage non fixé en ajoutant un réactif de capture comprenant des protéines d'immunoglobuline ou des fragments de celles-ci ; et
d) répéter en option lesdites étapes a), b) et c) pour former un groupe de sous-ensembles de complexes immuno-marqués, dans lequel chaque sous-ensemble se distingue d'un autre sous-ensemble par i) un rapport entre traceur et réactif de marquage ou ii) une propriété physique dudit traceur ou iii) un rapport entre réactif de marquage et ledit anticorps de liaison à une cible ou iv) par ledit anticorps de liaison à une cible,
dans lequel le procédéest **caractérisé en ce que** ledit réactif de marquage est un fragment Fab ou Fab' anti-région Fc.

2. Procédé selon la revendication 1, dans lequel ledit anticorps de liaison à une cible est un anticorps monoclonal murin, un anticorps polyclonal de lapin ou un anticorps polyclonal de chèvre.

3. Procédé selon la revendication 2, dans lequel lesdits anticorps de liaison à une cible sont dans une solution comprenant des protéines sériques ou des protéines d'ascites.

4. Procédé selon l'une quelconque des revendications 1 à 3, dans lequel ledit traceur est sélectionné parmi le groupe constitué d'un chromophore, d'un fluorophore, d'une protéine fluorescente, d'un colorant phosphorescent, d'un colorant tandem, d'une particule, d'un haptène, d'une enzyme et d'un radio-isotope.

5. Procédé selon la revendication 4, dans lequel ledit fluorophore est sélectionné parmi le groupe constitué d'une coumarine, d'un xanthène, d'une cyanine, d'un pyrène,d'un borapolyazaindacène, d'une oxazine et de dérivés de ceux-ci.

6. Procédé selon la revendication 4, dans lequel ladite protéine fluorescente est une phycobiliprotéine.

7. Procédé selon la revendication 4, dans lequel ledit colorant tandem est sélectionné parmi le groupe constitué d'un dérivé de cyanine-phycobiliprotéine et d'un dérivé de xanthène-phycobiliprotéine.

8. Procédé selon la revendication 4, dans lequel ladite enzyme est sélectionnée parmi le groupe constitué d'une peroxydase, d'une phosphatase, d'une glycosidase et d'une luciférase.

9. Procédé selon la revendication 4, dans lequel ladite particule est une microsphère ou une boîte quantique.

10. Procédé selon l'une quelconque des revendications précédentes, dans lequel ledit procédé comprend en outre la répétition des étapes a), b) et c) pour former des sous-ensembles de complexes immuno-marqués, dans lequel chaque sous-ensemble se distingue d'un autre sous-ensemble par i) un rapport entre traceur et réactif de marquage ou ii) des propriétés physiques dudit traceur ou iii) un rapport entre réactif de marquage et ledit anticorps de liaison à une cible ou iv) par ledit anticorps de liaison à une cible.
